# EUROPEAN PATENT SPECIFICATION

(11) **EP 2 909 632 B1**
(45) Date of publication and mention of the grant of the patent: **24.11.2021**
(21) Application number: 13783283.8
(22) Date of filing: 17.10.2013
(51) Int. Cl.: G01N 33/68

(54) **IMPROVED METHOD OF MAPPING GLYCANS OF GLYCOPROTEINS**
VERBESSERTES VERFAHREN ZUM MAPPING VON GLYCANEN UND GLYCOPROTEINEN
PROCÉDÉ AMÉLIORÉ DE MISE EN CORRESPONDANCE DE GLYCANES DE GLYCOPROTÉINES

(30) Priority: 17.10.2012 EP 12188904
(43) Date of publication of application: 26.08.2015
(73) Proprietor: Hexal AG, 83607 Holzkirchen (DE)
(72) Inventor: HIGEL, Fabian, 82041 Oberhaching (DE); SEIDL, Andreas, 82041 Oberhaching (DE)
(74) Representative: Dörries, Hans Ulrich
(86) International application number: PCT/EP2013/071788
(87) International publication number: WO 2014/060551

(56) References cited:
- WO-A1-2006/114663
- WO-A1-2012/124609
- US-A1- 2013 310 552
- BIGGE J C ET AL: "Nonselective and efficient fluorescent labelling of glycans using 2-amino benzamide and anthranilic acid", ANALYTICAL BIOCHEMISTRY, ACADEMIC PRESS INC, NEW YORK, vol. 230, no. 2, 1 January 1995 (1995-01-01), pages 229-238, XP002489980, New York NY USA ISSN: 0003-2697, DOI: 10.1006/ABIO.1995.1468
- Sigma: "Technical Bulletin: GlycoProfile 2-AA Labeling kit", 1 April 2005 (2005-04-01), Sigmq-Aldrich Inc., St louis MI USA, XP055055920, pages 1-4, page 3, section "Analysis of 2-AA labeled glycans"; page 4, section "references", nrs 1-8

## Description

### Field of the Invention

The present invention relates to the determination of glycosylation patterns in glycoproteins, e.g., in antibodies. Specifically, the present invention relates to methods of labeling the glycans of glycoproteins such as antibodies with anthranilic acid (2-AA) and separating the labeled glycans using reversed-phase liquid chromatography (RP-LC), particularly RP-LC under acidic conditions, followed by mass spectrometry (MS). The invention also relates to the use of anthranilic acid (2-AA) to label glycans of glycoproteins, e.g., of antibodies, prior to separation using reversed-phase liquid chromatography (RP-LC), particularly RP-LC under acidic conditions, again followed by MS. The present invention avoids the need for an ion-pairing reagent and is especially useful for the separation of neutral and acidic N-glycans of glycoproteins. The present invention may *inter alia* be used with reversed phase nano-liquid chromatography-mass spectrometry (RP-nano-LC-MS) technology. This allows for the use of 96-well plate sample preparation. It also provides high sensitivity (in the attomolar range) and the possibility to operate with high throughput.

### Background of the Invention

Biological drugs belong to the most complex pharmaceuticals. One important class of biological drugs are glycoproteins, and in particular monoclonal antibodies (mAbs), which are typically glycoproteins with a molecular mass of about 150 kDa. In recent years the glycosylation of therapeutic proteins came into focus as several investigations showed that these structures may have an effect on the safety and efficacy of the corresponding biological drugs.

IgGs have one conserved N-glycosylation site on each heavy chain at their Fc part, usually around amino acid 297. The heterogeneity of the N-glycans attached to these sites is very high and more than a dozen different glycans can be found. Identification and quantification of glycans from this mixture is sophisticated and demands a comprehensive analytical approach. Structural isomers of several glycans make the discrimination even more difficult.

N-glycosylation is a critical issue for the safety and efficacy of biopharmaceuticals. Hence, comprehensive analysis of N-glycosylation is necessary. N-glycosylation can be studied after enzymatic release of the glycans by PNGaseF using liquid chromatography, mass spectrometry or by a combination of both technologies (Chen and Flynn, Analytical Biochemistry 2007, 370, 147-61). Structural analysis of underivatized or labeled N-glycans is usually done by matrix-assisted laser desorption/ionization (MALDI-MS) or by porous graphitized carbon (PGC) liquid chromatography and on-line electrospray ionization mass spectrometry (ESI MS/MS).

Underivatized N-glycans have no light absorbing properties, so information gained from this kind of analytical methods is limited to mass spectrometry (MS) data. To circumvent these limitations, the N-glycans are often derivatized with fluorescence labels. For quantitative analytics of N-glycans, labeling with a fluorescence dye through reductive amination is widely used, offering high sensitivity. Every N-glycan carries only one label independent of size or branching, therefore, quantitative information can be obtained from the fluorescence signal intensity. The tag can not only provide UV or fluorescence detection but also the efficiency in electrospray ionization can be improved. For the analysis of sialic acid-containing glycans, labeling with a negatively charged tag, like 2-aminobenzoic acid (2-AA) or 8-aminonaphthalene-1,3,6-trisulfonic acid (ANTS), and MS detection in negative ionization mode is frequently used (Prien et al., Analytical Chemistry 2010, 82, 1498-508).

From the multitude of available fluorescence tags, 2-aminobenzamide (2-AB) is very popular and frequently used. A widely applied method is the separation and quantitation of 2-AB labeled N-glycans on a normal phase HILIC (Hydrophilic Interaction Liquid Chromatography) column. HILIC offers very good resolution and separation of many glycan isomers. However, the small injection volume of aqueous samples leads to a lowered sensitivity. Therefore, alternative approaches were developed like the use of reversed phase chromatography together with ESI-MS (Electrospray Ionization-Mass Spectrometry).

Since RP mobile phases usually only consist of water, an organic solvent and an acid, they are highly MS compatible. In addition there are almost no limitations with respect to injection volumes offering high sensitivity. It has been shown that RP-LC-MS of 2-AB, 2-AA or ANTS derivatized oligosaccharides can be performed (Chen and Flynn, Analytical Biochemistry 2007, 370, 147-61; Prien et al., Analytical Chemistry 2010, 82, 1498-508; Prater et al., Analytical Biochemistry 2009, 385, 69-79). For example, RP-HPLC (reversed-phase high performance liquid chromatography) coupled with ESI-MS has been used for the analysis of 2-AB-labeled N-glycans, including those released from a recombinant IgG antibody (Chen and Flynn, Analytical Biochemistry 2007, 370, 147-61). However, the method is not very selective for isomers and is time consuming. If 2-AB is used as fluorescence tag, only weak hydrophobicity is provided to the hydrophilic glycan, which results in a shallow and long chromatography gradient. Labeled acidic glycans elute early and are poorly separated. This problem was addressed in the art by the addition of an ion pairing reagent which increases the pH and thereby lowers the ionization efficiency (Melmer et al., Journal of Chromatography 2011, A 1218, 118-23).

Negatively charged labeling, in combination with negative ionization in MS, has been frequently used to analyze acidic N-glycans (Prien et al., Glycobiology 2010, 20, 629-47). For the characterization of oligomannose structures, 2-AA derivatized N-glycans were analyzed by a combination of reversed phase chromatography and negative mode mass spectrometry (Prien et al., Glycobiology 2010, 20, 629-47). According to this approach, N-glycans labeled with 2-AA can be separated using resolution reversed-phase separation with acetic acid-based mobile phases. However, separation of other isomers like G1F isomers with 1,3 and 1,6 galactosylation, which are highly abundant in most mAbs, has not previously been shown in any N-glycan reversed phase chromatography approach.

Also, prior to the present invention, an efficient and sensitive analysis of neutral and acidic N-glycans by RP-LC-MS in one approach was not available, since acidic 2-AB N-glycans elute in broad peaks at the beginning of the chromatographic run. Previous methods have been mostly limited to the isomer separation of oligomannose type N-glycans.

The inventors have now surprisingly found that the use of 2-AA for labeling glycoprotein glycans may overcome these disadvantages. Specifically, the inventors have found that 2-AA labeling of glycoprotein glycans is advantageous where the glycans are thereafter separated via RP-LC using an acidic mobile phase and subsequently analyzed by MS. Without intending to be bound by theory, it appears that at the low pH of the mobile phase of the RP-LC the carboxyl group of the 2-AA label is protonated and, hence, neutral, thereby resulting in a consequential higher hydrophobicity on the reversed phase column, which in turn leads to stronger retention of the 2-AA labeled N-glycans compared to 2-AB labeled glycans.

Also, the use of 2-AA as glycan label leads to efficient ionization in positive ionization mode of the mass spectrometer when the separated labeled glycans are subsequently analyzed via MS.

Furthermore, high injection volumes are possible when using the method of the invention, which means that comparatively large samples (e.g., 100 µl) can be injected into the HPLC system without further preparative steps after sample preparation, e.g., by gel-filtration. This is advantageous compared to the widely established HILIC method where only a few µl can be injected or the sample has to be concentrated and diluted in acetonitrile to enable higher injection volumes, hence enabling high sensitivity.

In addition, unlike the published approaches, there is no need for an ion pairing reagent to analyze acidic and neutral glycans in one run (i.e., when MS is directly coupled to, or "on-line" with, the RP-LC). An ion-pairing reagent (e.g., trifluoroacetic acid; TFA) is an amphiphilic molecule able to make hydrophobic as well as electrostatic interactions with the column surface and the polar analytes, respectively. When MS is directly coupled to the RP-LC one would expect a need for such a reagent to improve retention, e.g., of sialylated glycans. Surprisingly, it has been found that an ion-pairing reagent is not required in the context of the present invention. The resulting analytes elute in sharper peaks meaning at higher concentration and better sensitivity. This also allows for a reduced LC run time.

The ionization efficiency in positive electrospray ionization (ESI) is good for neutral as well as for acidic N-glycans in combination with the used acidic mobile phase.

The methods of analyzing glycoprotein glycans described and/or claimed herein, and the corresponding uses, thus offer high sensitivity and high resolution. The Examples demonstrate the feasibility of 2-AA as a label for the analysis of neutral and acidic N-glycans of antibodies with positive ESI MS. Compared to RP-LC-MS of 2-AB labeled N-glycans more structural isomers can be separated. The inventors have furthermore found that, surprisingly, the methods described and/or claimed herein may also be advantageously applied to other glycoproteins, e.g., to erythropoietin. Specifically, the methods described and/or claimed herein were found to be effective in separating erythropoietin glycans, which belong to the most complex glycans to be found in therapeutic glycoproteins.

Due to the high resolution of the liquid chromatography the high complexity of glycoprotein glycosylation can be investigated in detail. Through the high injection volume even low abundant N-glycans can be detected and quantified. The selectivity of the reversed phase chromatography separates various structural isomers of different types of N-glycans and more importantly separates the N-glycans in seven different groups, oligomannose, hybrid and complex glycans without core fucosylation and hybrid and complex glycans with core fucosylation as well as two sialic acid containing groups again with and without the core fucose residue.

Where information about the N-glycan linkages cannot be deduced by the method of the invention enzymatical digestion with exoglucosidases may be performed to discriminate between several isomers. Hybrid structures with differences in the hexose number can thus be solved.

Apart from the afore-mentioned advantages of 2-AA as glycan label compared to, e.g., 2-AB (including stronger retention on the RP-column during RP-LC compared to the use of 2-aminobenzamide (2-AB) and efficient ionization and detection of 2-AA labeled N-glycans during MS detection while allowing decreased LC run time), the inventors have also found that the acidic conditions during RP-LC lead to an efficient separation of acidic 2-AA N-glycans carrying terminal sialylation without the need for an ion-pairing reagent. 2-AA N-glycans are more sensitive in fluorescence detection and better ionization is achieved compared to methods using 2-AB. Moreover, in contrast to HILIC, no buffered mobile phase containing ammonium is used. This is an additional advantage since ammonium can suppress efficient ionization.

The method of the present invention can be used, for example, to characterize the glycosylation of antibodies or other glycoproteins in detail in a single analytical approach. The novel approach described and claimed herein can be used to create a comprehensive glycan map with the ability to detect and identify even minor portions of the N-glycan pool of a given glycoprotein, such as a monoclonal antibody (mAb).

Characterization of N-glycosylation is of importance during biopharmaceutical process development because N-glycosylation may affect safety or efficacy of a protein drug (Chung et al., The New England Journal of Medicine 2008, 358, 1109-17; Morell et al., The Journal of Biological Chemistry 1971, 246, 1461-7; Shields et al., The Journal of Biological Chemistry 2002, 277, 26733-40; Jefferis, Nature Reviews, Drug Discovery 2009, 8, 226-34; and Liu et al., Journal of Pharmaceutical and Biomedical Analysis 2011, 54, 27-36). For monoclonal antibodies these effects are based on structural properties derived from the CH2 domain glycosylated at Asn at the approximate position Asn²⁹⁷. Size and charge of attached N-glycans as well as terminal sugar moieties influence e.g. CDC and ADCC potency of IgGs and thereby the overall efficacy. For example, lack of core fucose increases ADCC by improving binding to FcyRllla. Increased ADCC activity could be correlated with product safety, i.e., serious infections during TNFα treatment in rheumatoid arthritis patients (Favalli et al., Autoimmunity Reviews 2009, 8, 266-73). Lack of terminal galactose residues and resulting terminal GlcNAc residues increases CDC by modulating binding to C1q (Raju, Current Opinion in Immunology 2008, 20, 471-8). Therefore, it is crucial to analyze the glycan pattern of a biopharmaceutical as early as possible during development to be able to modify the drug candidate, for example by glyco-engineering. Alterations of IgG N-glycosylation have been linked with aging and a variety of diseases and distinct N-glycans are regarded as potential biomarkers as the above described interactions of IgGs and Fc-receptors influence and finally modulate immune responses (Abd Hamid, Glycobiology 2008, 18, 1105-18; Ruhaak et al., Analytical Chemistry 2008, 80, 6119-26; Bones et al., Analytical Chemistry 2010, 82, 10208-15; Ruhaak et al., Journal of Proteome Research 2011, 10, 1667-74; Malhotra et al., Nature Medicine 1995, 1, 237-243; Yamada et al., Glycoconjugate Journal 1997, 14, 401-5; Wuhrer et al., Proteomics 2007, 7, 4070-81; and Kodar et al., Glycoconjugate Journal 2012, 29, 57-66).

WO 2006/114663 A1 describes a method of determining one or more glycosylation markers of disease and a method for diagnosing and monitoring disease in a subject comprising obtaining a sample of body fluid or a body tissue of the subject; releasing a glycan pool and measuring a glycoprofile of the glycan pool.

Bigge JC, et al., 1995 (Analytical Biochemistry. Academic Press Inc., New York; 230(2):229-238) discloses reaction conditions for the conjugation of 2-AB and 2-AA to N- and O-glycans and the Sigma "Technical Bulletin: GlycoProfile™ 2-AA Labeling kit" (1 April 2005, Sigma-Aldrich Inc., St. Louis, MI, USA) describes the GlycoProfile™ 2-AA Labeling kit and how to use it for labeling glycans.

N-glycosylation analysis is sophisticated because of countless N-glycan variants which may be attached to the protein molecules and the huge differences in their relative amounts. For example, for recombinant human IgG antibodies relative amounts of individual oligomannose N-glycans may range from 0.02% to more than 70% for the most abundant N-glycan, differences that cover three orders of magnitude (Higel et al., Analytical and Bioanalytical Chemistry 2013, 405, 2481-93). Technologies frequently used for N-glycan analysis are CE, HPAEC-PAD, HPLC, MALDI and ESI-MS and various combinations of these technologies (Mariño et al., Nature Chemical Biology 2010, 6, 713-23). LC-MS is an advantageous combination as LC is able to separate glycan mixtures; afterwards glycan variants can then individually be identified and quantified by on-line MS. For various analytical applications, however, conventional LC-MS has insufficient sensitivity, especially for cases where the sample amount is strongly limited. During early biopharmaceutical development, clone or pool selection, only minute amounts of recombinant protein from micro titer plates are usually available for protein and glycan analysis.

N-glycan biomarker discovery in patients or healthy individuals is another scenario where sample amount is typically very limited. In proteomics, limitations of this kind have been circumvented by reducing the dimensions of the analytical system, for example, by the use of nano-LC-MS. Approaches for N-glycan analysis by the use of nano-LC-MS are rare in the literature. Three investigations have reported feasibility of nano-LC for glycan analysis. Wuhrer *et al.* have miniaturized HILIC-MS to nanoscale for oligosaccharide analysis (Wuhrer et al., Analytical Chemistry 2004, 76, 833-8). They have analyzed underivatized N-glycans with femtomolar sensitivity. Avoiding glycan derivatization shortens sample preparation but the benefit of improved MS detection coming with the label is lost (Wuhrer et al., Analytical Chemistry 2004, 76, 833-8; Ruhaak et al., Analytical and Bioanalytical Chemistry 2010, 397, 3457-81). Kalay *et al.* have used normal phase nanoscale HPLC-MS with on-line fluorescence to analyze 2-AB N-glycans (Analytical Biochemistry 2012, 423, 153-162). Their approach resulted in long and time consuming gradients to achieve a good chromatographic resolution. One work utilizing nano-reversed phase chromatography (nano-RPC) has been published recently. Ritamo et al. (Analytical and Bioanalytical Chemistry 2013, 405, 2469-80) have used a nano-LC system to separate permethylated N-glycans. They have achieved separation for various structural isomers on a nano-RP-column. Permethylation of N-glycans, however, is complex and toxic reagents are used and formation of side products is rather likely which makes routine use questionable. It has been reported previously that RP-LC with on-line MS is broadly applicable for analysis of differently reducing-end labeled N-glycans (Chen and Flynn, Analytical Biochemistry 2007, 370, 147-61; Prater et al., Analytical Biochemistry 2009, 385, 69-79).

It has been found that the methods and uses of the present invention may be practiced using RP-nano-LC-MS, thereby offering the option of utilizing a 96-well based sample preparation work-flow. These methods and uses are simple, offer high throughput, and can be used routinely, e.g., during early biopharmaceutical development or to discover clinical relevant changes of N-glycosylation with high sensitivity. The method has high resolving power, is selective for many N-glycan structural isomers, including multiple branched and acidic variants, and, in addition, 2-AA labeled glycans can be identified by MSⁿ with the use of ion-trap mass spectrometry. This allows an overall sensitivity for a single N-glycan on column of, e.g., about 400 attomole with the use of a recently introduced MS source.

The approach according to this aspect of the invention is particularly useful during early biopharmaceutical development where clone or pool selection is often involved, since only minute amounts of recombinant protein (e.g., antibody) from microtiter plates are usually available for protein and glycan analysis. N-glycan biomarker discovery in patients or healthy individuals is another scenario where this approach would be advantageous, since sample amount is typically very limited. This is shown by comparing the N-glycosylation of Fc containing therapeutic protein from different cell clones and the broad applicability is demonstrated by glycan analysis of IgGs from human serum. The approach is versatile, and due to the use of reversed phase chromatography (RPC), it will be applicable in many analytical laboratories already working with nano-LC-MS with little effort.

### Summary of the Invention

The scope of protection is defined by the appended claims. Hereinafter, the terminology, "embodiment" relates to the disclosure and only form part of the invention if it falls within the scope of the claims.

Accordingly, the present invention relates to a method of analyzing the glycans of a glycoprotein, comprising labeling the glycans with anthranilic acid (2-AA); and separating the labeled glycans using reversed-phase liquid chromatography (RP-LC). The RP-LC is performed with a mobile phase that is acidic. Alternatively, or in addition, it is performed under conditions under which the carboxyl group of the 2-AA label attached to the glycans is neutral. Additionally, no ion-pairing reagent is used in the mobile phase. In a preferred embodiment, the method further comprises subjecting the 2-AA labeled glycans after their separation via RP-LC to mass spectrometry (MS). Particularly preferred are methods where this is done by directly coupling RP-LC with the MS analysis such that there is no further analytical or preparatory step between the RP-LC and the MS analysis. Alternatively, however, other analytical or preparatory step may well be included between the RP-LC and the MS analysis step.

The present invention furthermore relates to the use of 2-AA as a label for the glycans of a glycoprotein in the method of analysing according to claim 1.

The present disclosure also relates to a method of preparing a glycoprotein based pharmaceutical composition comprising analyzing the glycans of said glycoprotein by any of the methods or uses described and or claimed herein and formulating the glycoprotein into said pharmaceutical composition.

Likewise encompassed by the present disclosure is a glycoprotein, and more particularly an antibody, comprising a glycan labeled with 2-AA.

In a preferred embodiment, the RP-LC is RP-nano-LC. In one aspect the glycoprotein is derived from one or more protein-producing higher eukaryotic cell clones, wherein the clone is optionally producing an antibody. In another embodiment, the glycoprotein is derived from one or more samples from one or more patients or subjects, wherein the one or more samples is optionally human serum. In a particular aspect the glycoprotein (e.g., derived from a clone or serum) is applied to a 96-well filter-plate well containing Protein A or Protein G sepharose.

As noted above, the present disclosure allows the analysis of anthranilic acid labeled glycans, particularly N-glycans, with high sensitivity and selectivity. The methods and uses according to the disclosure allow the analysis of acidic and neutral N-glycans in one approach, using a highly MS compatible mobile phase for liquid chromatography. The mass spectrometer can be operated in positive ionization for both neutral and acidic N-glycans

The use of anthranilic acid according to the invention to label N-glycans with a more acidic mobile phase is advantageous compared to the use of 2-AB in RP-LC. At lower pH values, the carboxylic acid group of2-AA is protonated, and thus, neutral, thus having a higher hydrophobicity and therefore a stronger retention on the reversed phase column. The on-line mass spectrometry detection with positive ionization is also favored under acidic conditions due to the high proton concentration. This allows efficient ionization and detection of 2-AA labeled N-glycans. The acidic conditions also lead to an efficient separation of acidic 2-AA N-glycans carrying terminal sialylation, without the need of an ion-pairing reagent.

The use of formic acid instead of, e.g., acetic acid, as a component of the mobile phase, further improves the separation. The low pH of the mobile phase achieved with the formic acid leads to an improved retention of the 2-AA labeled N-glycans and to an efficient ionization in positive ionization mode of the mass spectrometer.

The present disclosure provides high selectivity for N-glycan isomers for all types of N-glycans (oligomannose, hybrid and complex). 2-AA provides better separation in RP-HPLC that allows shorter run times on a RP-column. This allows positive electrospray ionization mass spectrometry (ESI-MS) of both neutral and acidic glycans and acidic glycans to elute according to their family. Even sialic acid containing glycans are easily detected with high intensity. For example, effective separation of the highly abundant G1F isomers of mAbs is achieved.

### Brief Description of the Figures

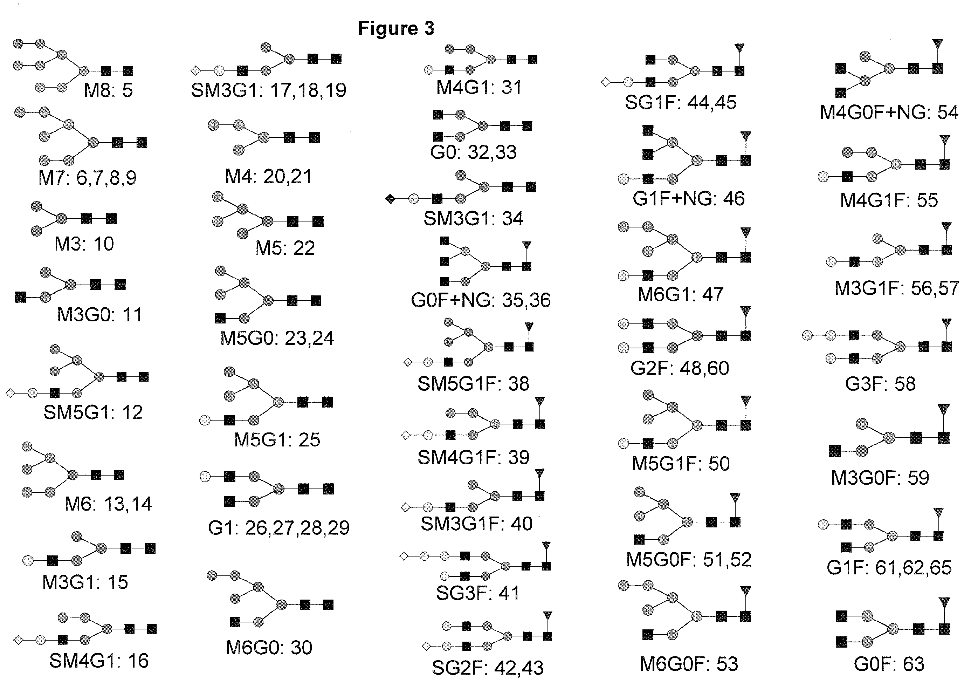

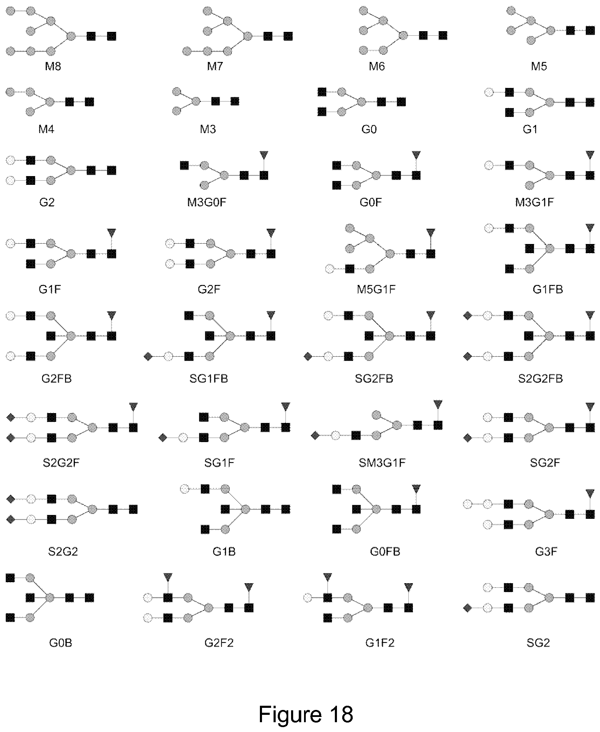

**Figure 1**
   Fluorescence chromatogram of 2-AB N-glycans separated on a RP-column. Zoomed view (B) of the chromatogram shows the smaller peaks of less abundant N-glycans. The numbered peaks were identified using MS and MS². Stacked numbering indicates co-elution of N-glycans. Table 1 lists the MS data of identified peaks. The 2-AB glycans elute in different groups according to their type. Oligomannose glycans elute first, followed by acidic hybrid and complex type as well as neutral hybrid glycans. Acidic hybrid with core fucose elute after complex glycans. Neutral hybrid structures co-elute between approx. 57 and 86 min with acidic complex followed by complex 2-AB glycans, all three groups carrying a core fucose.
**Figure 2**
   RP-chromatogram of the mAb glycan standard showing the grouping of the eluting 2-AA N-glycans. (A) High Mannose structures elute first, followed by non-fucosylated hybrid and complex glycans. Fucosylated hybrid and complex structures elute last in the chromatogram. Acidic glycans elute right before their appropriate neutral glycans. (B) Zoom of the region between 18 and 42 min showing the less abundant glycans. The identified glycans are numbered and the appropriate masses are listed in Table 1. Stacked numbering indicates co-elution of N-glycans. The glycan structures are depicted in Figure 3.
**Figure 3**
   Symbol structures of the identified N-glycans. The numbering is according to the peak numbering for the N-glycans in the fluorescence chromatograms and in Table 1. For structures with multiple peak assignments only one possible isomer is drawn. Symbols: ▼ L-Fucose, ■ N-Acetyl-D-glucosamine, 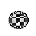 D-Mannose, 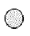 D-Galactose, ◇ N-Glycoyl-neuraminic acid, ◆ N-Acetyl-neuraminic acid.
**Figure 4**
   MS² spectrum of the 2-AA labeled G1 FSG glycan from mAb3 at *m*/*z* 1027.9. The dissociated bonds of the [M+2H]²⁺ ion are depicted and the assigned B and Y ions are labeled in the spectrum.
**Figure 5**
   MS² spectrum of the 2-AA labeled M5G1FSG glycan from mAb3 at *m*/*z* 1088.4. Resulting single charged B and Y ions from the [M+2H]²⁺ ion are shown. B ions were exclusively from the α-branch containing a GlcNAc (N-acetyl-D-glucosamine) that is able to carry a charge.
**Figure 6**
   MS² spectrum of the 2-AA labeled G3F N-glycan from mAb3. The dissociated bonds of the [M+2H]²⁺ ion are depicted and the assigned B and Y ions are labeled in the spectrum. The glycan accounts for <0.01% of the glycan pool of mAb3.
**Figure 7**
   EICs of four structural M7 isomers from mAb2. Panel A shows the EIC of 2-AB labeled N-glycans. Panel B shows the EIC for the 2-AA labeled glycans. The selectivity is identical for the M7 isomers in both approaches.
**Figure 8**
   EICs of the G1F glycan from mAb2. The 2-AB labeled G1F elutes in one peak (A), whereas the 2-AA labeled glycans (B) are separated into the two isomers. The terminal galactose residue (C) can either be linked to the a1,6 or the a1,3 branch of the bi-antennary N-glycan.
**Figure 9**
   RP FLD chromatogram of the mAb1 N-glycan pool after labeling with 2-AA. Peaks identified by MS and MS² are numbered and listed in Table 1. The appropriate 2-AA glycans are shown in Table 1. A zoomed view (small window) of the chromatogram shows the smaller peaks.
**Figure 10**
   RP FLD chromatogram of the mAb2 N-glycan pool after labeling with 2-AA. Peaks identified by MS and MS² are numbered and listed in Table 1. The appropriate 2-AA glycans are shown in Figure 3. A zoomed view (small window) of the chromatogram shows the smaller peaks. Stacked numbering indicates co-elution of N-glycans.
**Figure 11**
   RP FLD chromatogram of mAb3 N-glycan pool after labeling with 2-AA. Peaks identified by MS and MS² are numbered and listed in Table 1. The appropriate 2-AA glycans are shown in Figure 3. A zoomed view (small window) of the chromatogram shows the smaller peaks. Stacked numbering indicates co-elution of N-glycans.
**Figure 12**
   Extracted ion chromatograms of 2-AA labeled N-glycans derived from Ribonuclease B (RNAse B). Peaks are numbered by elution order. Identified glycans are listed in Table 2.
**Figure 13**
   Analysis of highly acidic and multiply branched N-glycan standards. Overlay of EICs from different 2-AA labeled glycan standards is shown. Each standard was analyzed individually. N-glycan structures corresponding to the peak numbering are depicted on the left. For N-glycans with several structural isomers one possible structure is shown.
**Figure 14**
   Schematic work-flow. Several 96 well samples can be handled simultaneously. Immobilized Protein A or Protein G is used to capture mAbs, Fc part containing fusion proteins or other IgGs with high specificity. Immobilized target is then highly efficiently deglycosylated with the use of PNGaseF. Released N-Glycans are labeled with 2-AA via reductive amination after ultrafiltration to remove remaining proteins. Labeled and purified 2-AA glycans are identified and quantified by RP-nano-LC-MS by use of an ion-trap mass spectrometer.
**Figure 15**
   Determining linearity and limits of the method by use of 2-AA labeled G0F standard in a serial dilution. (A) EICs of injections from 2200 fmol to 2 fmol. Peaks had a constant retention time around 55 minutes. (B) Magnified view of the four lowest amounts. (C) Peak areas of EICs are plotted against the glycan amount on column to show the linear correlation. 800 amol was the lowest amount that fitted the linear regression. 600 and 400 amol were also detectable, but did not fit linear regression. 800 amol is the lowest amount for reliable quantification, the lower limit of quantification. (D) Magnified view of four lowest injection amounts (22 fmol - 0.8 fmol) that fitted into the linear regression.
**Figure 16**
   MS spectra of high and low G0F 2-AA dilutions. (A) MS spectrum of 1.1 pmol injection showing the [M+H]²⁺ ion at 792.7 m/z. (B) MS spectrum of 2.2 fmol injection. [M+H]²⁺ ion at 792.7 m/z is clearly visible with good S/N. (C) Exemplarily isotopic pattern of double charged 2-AA G0F at 792.7 m/z.
**Figure 17**
   Nano-LC-MS Glycan Map of a monoclonal antibody. 1.6 pmol of 2-AA labeled N-glycans corresponding to approximately 120 ng antibody were injected. EICs of the 2-AA labeled N-glycans are depicted. Complete glycan map (A) and magnified view without the three most abundant glycans (B) are shown. Peaks are numbered in elution order. Identified glycans are listed in Table 5, glycan structures are depicted in Figure 18.
**Figure 18**
   Glycan structures found in serum IgGs and the monoclonal antibody. **Symbols: ■ N-Acetylglucosamine,** 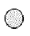 **Galactose, • Mannose, ▼ Fucose, ◆ N-Acetylneuraminic Acid.** For glycan structures with several isomers only one possible structure is depicted exemplarily.
**Figure 19**
   Glycan Map of two Fc containing therapeutic proteins derived from clone selection phase. Clone 1 (upper panel) and clone 2 (lower panel) are shown exemplarily. EICs of the different glycoforms are shown. Glycosylation pattern is similar for both clones. Glycans 14 and 15 are only present in clone 2 (lower panel). Glycan structures are depicted in Figure 18 according to peak numbering. Quantitative data is listed in Table 5.
**Figure 20**
   Glycan map of human serum IgGs. (A) EICs of the various 2-AA labeled N-glycans are shown. Peak numbering is according to elution order. Table 6 lists the identified glycans and their relative amount and Figure 18 shows the glycan structures.
**Figure 21**
   MS² spectra of two glycans each carrying two fucose residues. (A) MS² of G2F2. Precursor ion was double charged 1027.8 m/z. The glycan can be identified unambiguously due to two signature fragments at 1689 m/z and 512 m/z respectively. (B) MS² of G1F2. Precursor ion was double charged 946.8 m/z. This glycan can be identified according to the fragments at 512 m/z and 1527 m/z.

### Detailed Description of the Invention

### Definitions

As used herein, 2*-AA* is 2-amino benzoic acid, also known as anthranilic acid. For the purposes of the present invention 2-AA includes isotopic 2-AA, for example, the stable isotopic ¹²[C₆]-2-AA) or ¹³[C₆]-2-AA variants (Prien, J.M., Prater, B.D., Qin, Q. & Cockrill, S.L., Mass spectrometric-based stable isotopic 2-aminobenzoic acid glycan mapping for rapid glycan screening of biotherapeutics, Analytical Chemistry 82, 1498-508 (2010)). Glycans labelled with such isotopic variants of 2-AA may be analyzed using positive-mode matrix-assisted laser desorption ionization time-of-flight mass spectrometry (MALDI-TOF MS) or on-line negative-mode electrospray mass spectrometry (ESI-MS/MSⁿ) and/or nanospray mass spectrometry (NSI-MS) (Prien, J.M., Prater, B.D., Qin, Q. & Cockrill, S.L., Mass spectrometric-based stable isotopic 2-aminobenzoic acid glycan mapping for rapid glycan screening of biotherapeutics, Analytical Chemistry 82, 1498-508 (2010)).

Carbohydrate moieties are described herein with reference to commonly used nomenclature for oligosaccharides. A review of carbohydrate chemistry which uses this nomenclature can be found, for example, in Hubbard and Ivatt, Ann. Rev. Biochem. 50, 555-583 (1981).

For the purposes of the present disclosure a *glycan* refers to any sugar or assembly of sugars, in free form or attached to another molecule, (i.e., saccharide or carbohydrate). As referred to herein, an N-*glycan* is a glycan covalently linked to an asparagine residue of a polypeptide chain in the consensus sequence: -Asn-X-Ser/Thr (e.g., Manβ1-4GlcNAcβ1-4GlcNAcβ1-N-Asn). As referred to herein, an *acidic glycan* is an N-glycan containing at least one terminal sialic acid (at the non-reducing terminus). As referred to herein, a *neutral glycan* is an N-glycan that does not contain any sialic acid. As referred to herein, *glycosylation* is the enzyme-catalyzed covalent attachment of a carbohydrate to a polypeptide, lipid, polynucleotide, carbohydrate, or other organic compound, generally catalyzed by glycosyltransferases, utilizing specific sugar nucleotide donor substrates. For the purposes of the present invention, a *polysaccharide* is a glycan composed of repeating monosaccharides, preferably greater than ten monosaccharide units in length. As referred to herein, a *sugar* refers to any carbohydrate, preferably to low molecular weight carbohydrates that are sweet in taste (see glossary of Essentials of Glycobiology. 2nd edition. Varki A, Cummings RD, Esko JD, et al., editors. Cold Spring Harbor (NY): Cold Spring Harbor Laboratory Press; 2009).

For the purposes of the present disclosure the term *glycoprotein* refers to peptides and proteins, including antibodies, having at least one glycan side chain. Preferred glycoproteins are glycan containing therapeutic proteins that have been expressed in higher eukaryotic cells. In this regard, exemplary useful higher eukaryotic cells are selected from the following cell lines:

| Cell Line | Meaning | Origin | Tissue Origin | Morphology |
|---|---|---|---|---|
| CHO | Chinese hamster ovary | Hamster | Ovary | Epithelium |
| COS-7 | *Cercopithecus aethiops,* origin-defective SV-40 | Ape-*Cercopithecus aethiops (Chlorocebus)* | Kidney | Fibroblast |
| BHK-21 | Baby hamster kidney fibroblast cells | Hamster | Kidney | Fibroblast |
| HEK-293 | Human embryonic kidney | Human | Kidney (embryonic) | Epithelium |
| HeLa | "Henrietta Lacks" | Human | Cervical cancer | Epithelium |
| HL-60 | Human leukemia | Human | Myeloblast | Blood cells |
| HUVEC | Human umbilical vein endothelial cell | Human | Umbilical vein endothelium | Epithelial |
| Jurkat | | Human | T cell leukemia | white blood cells |
| MCF-7 | Michigan Cancer Foundation-7 | Human | Mammary gland | Invasive breast ductal carcinoma |
| NIH-3T3 | NIH, 3-day transfer, inoculum 3 x 10⁵ cells | Mouse | Embryo | Fibroblast |
| RenCa | Renal carcinoma | Mouse | | Renal carcinoma |
| U937 | | Human | Leukaemic monocytic lymphoma | |
| Vero cells | *Vero* (truth) | African green monkey | Kidney epithelium | |

Particularly preferred in this regard are CHO cells, which are widely used in the art to express biopharmaceutically useful glycoproteins, such as antibodies.

The glycoproteins may be homologous to the host cell, or may be heterologous, i.e., foreign, to the host cell being utilized, such as, for example, a human glycoprotein produced by a Chinese hamster ovary (CHO) host cell. Such glycoproteins are generally referred to as *recombinant glycoproteins.*

In certain aspects the glycoproteins expressed by a host-cell are directly secreted into the medium.

Examples of suitable mammalian, and in particular human, glycoproteins include the following molecules: a cytokine; a cytokine receptor; a chemokine, such as TNF and TECK; a chemokine receptor, such as a TNFR and CCR9; a growth hormone, such as human growth hormone and bovine growth hormone; growth hormone releasing factor; parathyroid hormone; thyroid stimulating hormone; a lipoprotein; alpha-1-antitrypsin; insulin A-chain; insulin B-chain; proinsulin; follicle stimulating hormone; calcitonin; luteinizing hormone; glucagon; human macrophage inflammatory protein (MIP-1-alpha); a serum albumin, such as human serum albumin; mullerian-inhibiting substance; relaxin A-chain; relaxin B-chain; prorelaxin; mouse gonadotropin-associated peptide; a clotting factor, such as factor VIIIC, factor IX, tissue factor, and von Willebrand factor; an anti-clotting factor, such as Protein C; atrial natriuretic factor; lung surfactant; a plasminogen activator, such as urokinase or human urine or tissue-type plasminogen activator (t-PA); bombesin; thrombin; hemopoietic growth factor; enkephalinase; RANTES; a microbial protein, such as beta-lactamase; DNase; inhibin; activin; vascular endothelial growth factor (VEGF); a receptor for hormones or growth factors; an integrin; protein A or D; a rheumatoid factor; a neurotrophic factor such as bone-derived neurotrophic factor (BDNF), neurotrophin-3, -4, -5, or -6 (NT-3, NT-4, NT-5, or NT-6); a nerve growth factor, such as NGF-beta; platelet-derived growth factor (PDGF); a fibroblast growth factor, such as aFGF and bFGF; epidermal growth factor (EGF); a transforming growth factor (TGF) such as TGF-alpha and TGF-beta, including TGF-beta1, TGF-beta2, TGF-beta3, TGF-beta4, or TGF-beta5; insulin-like growth factor-I and -II (IGF-I and IGF-II); des(I-3)-IGF-l (brain IGF-I); an insulin-like growth factor binding protein, a CD protein, such as CD-3, CD-4, CD-8, and CD-19; erythropoietin; an osteoinductive factor, an immunotoxin; a bone morphogenetic protein (BMP); an interferon, such as interferon-alpha, -beta, and -gamma; a colony stimulating factor (CSF), such as M-CSF, GM-CSF, and G-CSF; an interleukin (ILs), such as IL-1 to IL-21; superoxide dismutase; a T-cell receptor; a cell surface membrane protein; a transport protein; a homing receptor; a regulatory protein; a decay accelerating factor; and a viral antigen, such as, a portion of the HIV-1 or HIV-2 envelope protein.

A preferred glycoprotein in the context of the present invention is a hemopoietic growth factor, such as erythropoietin, or a colony stimulating factor (CSFs), such as M-CSF, GM-CSF, and G-CSF. Particularly preferred in this regard is erythropoietin.

Another preferred glycoprotein in the context of the present invention is an antibody.

The term *antibody* as referred to herein includes whole antibodies and any antigen binding fragment (i.e., *antigen-binding portion*) or single chain thereof. An *antibody* refers to a glycoprotein comprising at least two heavy (H) chains and two light (L) chains inter-connected by disulfide bonds, or an antigen binding portion thereof. Each heavy chain is comprised of a heavy chain variable region (abbreviated herein as V_{H}) and a heavy chain constant region. The heavy chain constant region is comprised of three domains, CH1, CH2 and CH3. Each light chain is comprised of a light chain variable region (abbreviated herein as V_{L}) and a light chain constant region. The light chain constant region is comprised of one domain, C_{L}. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system.

Examples of antigen-binding fragments encompassed within the term *antigen-binding portion* of an antibody include (i) a Fab fragment, a monovalent fragment consisting of the V_{L}, V_{H}, CL and CH1 domains; (ii) a F(ab')2 fragment, a bivalent fragment comprising two Fab fragments linked by a disulfide bridge at the hinge region; (iii) an Fd fragment consisting of the V_{H} and CH1 domains; (iv) an Fv fragment consisting of the V_{L} and V_{H} domains of a single arm of an antibody, (v) a dAb fragment (Ward et al., Nature. 341:544-546 (1989)), which consists of a V_{H} domain; and (vi) an isolated complementarity determining region (CDR) or (vii) a combination of two or more isolated CDRs which may optionally be joined by a synthetic linker. Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); see e.g., Bird et al. Science 1988, 242:423-426; and Huston et al., Proc. Natl. Acad. Sci. USA 1988, 85:5879-5883). Such single chain antibodies are also intended to be encompassed within the terms *antigen-binding portion* and *antigen-binding fragment* of an antibody. These antibody fragments are obtained using conventional techniques known to those with skill in the art, and the fragments are screened for utility in the same manner as are intact antibodies.

The term *monoclonal antibody* (mAb), as used herein, refers to an antibody which displays a single binding specificity and affinity for a particular epitope. Accordingly, the term *human monoclonal antibody* refers to an antibody which displays a single binding specificity and which has variable and constant regions derived from human germline immunoglobulin sequences. In one embodiment, human monoclonal antibodies are produced by a hybridoma which includes a B cell obtained from a transgenic non-human animal, e.g., a transgenic mouse, having a genome comprising a human heavy chain transgene and a light chain transgene fused to an immortalized cell.

The term *recombinant human antibody,* as used herein, includes all human antibodies that are prepared, expressed, created or isolated by recombinant means, such as (a) antibodies isolated from an animal (e.g., a mouse) that is transgenic or transchromosomal for human immunoglobulin genes or a hybridoma prepared therefrom, (b) antibodies isolated from a host cell transformed to express the antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable and constant regions derived from human germline immunoglobulin sequences. In certain embodiments, however, such recombinant human antibodies can be subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

As used herein, a *heterologous antibody* is defined in relation to the transgenic non-human organism producing such an antibody. This term refers to an antibody having an amino acid sequence or an encoding nucleic acid sequence corresponding to that found in an organism not consisting of the transgenic non-human animal, and generally from a species other than that of the transgenic non-human animal.

As used herein, *specific binding, selective binding* and *selectively binds,* refer to an antibody or a fragment thereof, binding to a predetermined antigen. For example, in one embodiment, the antibody binds with an affinity (K_{D}) of approximately less than 10⁻⁷ M, such as approximately less than 10⁻⁸ M, 10⁻⁹ M or 10⁻¹⁰ M or even lower when determined by surface plasmon resonance (SPR) technology in a BIACORE 3000 instrument using an analyte and the corresponding antibody as the ligand, and binds to the predetermined antigen with an affinity that is at least two-fold greater than its affinity for binding to a non-specific antigen (e.g., BSA, casein) other than the predetermined antigen or a closely-related antigen. The phrases *an antibody recognizing an antigen* and *an antibody specific for an antigen* are used interchangeably herein with the term *an antibody which selectively binds to an antigen.*

As used herein, *reversed phase LC* or *HPLC (RP-LC* or *RP-HPLC)* has a non-polar stationary phase and an aqueous, moderately polar mobile phase, e.g., a silica which has been surface-modified with RMe₂SiCl, where R is a straight chain alkyl group such as C₁₈H₃₇ or C₈H₁₇ (Lehto and Hou, Chemistry and Analysis of Radionuclides, Wiley-VCH Verlag & Co., Weinheim, Germany, 2011, page 170).

As used herein *reversed phase nano-LC* or *nano-HPLC (RP-nano-LC or RP-nano-HPLC)* is characterized by a decreased inner diameter of the columns that are used for LC (10-150 µm) and smaller flow-rates (10-1000 nl/min) compared to conventional LC or HPLC, respectively. This down-scaling results in high plate counts of the nano-LC sytem and the ability to analyze proteinaceous samples in the low femtomole and subfemtomole ranges (Chervet et al., Analytical Chemistry 1996, 68:1507-12). Consistent with the understanding and common general knowledge in the field of liquid chromatography, it will be appreciated that in accordance with the invention nano-LC and nano-HPLC are suitable and intended forms of LC and HPLC, respectively, for the purposes of the present invention; and that RP-nano-LC and RP-nano-HPLC are suitable and intended forms of RP-LC and RP-HPLC, respectively. The same applies to the likewise well-known and established techniques of *micro-LC* and *capillary-LC,* or *RP-micro-LC* and *RP-capillary-LC,* which for the purposes of the present invention are suitable and intended forms of LC, and RP-LC, respectively.

For the purposes of the present invention, a *mobile phase* of RP-LC or RP-HPLC is preferably a gradient of an organic modifier (e.g., acetonitrile or methanol) in water, with an ionic modifier that controls the pH and ionization state or acts as an *ion pairing reagent.* Anionic ion-pair reagents (e.g., trifluoroacetic acid (TFA)) bind to protonated basic groups of peptides. The addition of 0.1% TFA acidifies the eluent which causes the carboxylic groups of peptides and proteins to become protonated, resulting in a larger hydrophobicity of the molecules. Cationic ion-pairing reagents (e.g., triethylammonium ions) bind to ionized carboxyl groups of peptides (*"*Protein Liquid Chromatography", Journal of Chromatography Library, vol. 61, edited by Kastner M, Elsevier Science B.V., 2000, page 153). Diethylamine (DEA) can also be used as an ion pairing reagent (Melmer et al., Journal of Chromatography A (2011), Volume: 1218(1): 118-123).

As used herein, *ion trap mass spectrometry* is an arrangement in which ions with a desired range of quotients mass/charge are first made to describe stable paths under the effect of a high-frequency electric quadrupole field, and are then separated and presented to a detector by adjusting the field so as to selectively induce path instability according to their respective mass/charge ratios e.g., quadrupole ion trap (see http://www.genomicglossaries.com/content/mass_spectrometry.asp).

For the purposes of the present invention, all *N-glycans* are understood to have the common core sugar sequence, Manα1―6(Manα1―3)Manβ1―4GlcNAcβ1―4GlcNAcβ-Asn-X-Ser/Thr, and are classified into three types: (1) *oligomannose*, in which only mannose residues are attached to the core; (2) *complex*, in which "antennae" initiated by N-acetylglucosaminyltransferases (GlcNAcTs) are attached to the core; and *(3) hybrid,* in which only mannose residues are attached to the Mana1-6 arm of the core and one or two antennae are on the Mana1-3 arm (Essentials of Glycobiology, 2nd edition, Varki et al., editors, Cold Spring Harbor (NY): Cold Spring Harbor Laboratory Press; 2009; Chapter 8).

For the purposes of the present invention, a reference to the analysis of acidic or neutral glycans, N-glycans, or glycans in general, *in one run*, *in one approach*, or *in a single (analytical) approach* means that the MS analysis is directly coupled to (or in other words, is performed on-line with) the RP-LC step. This means that there is no further analytical or preparatory step between the RP-LC and the MS analysis. It will be understood that this does not exclude that fluorescence detection will occur between the RP-LC and the MS. Indeed, fluorescence detection of the 2-AA labeled glycans separated on the RP-column will normally (and advantageously) occur before they leave the LC system and proceed to MS, since the fluorescence detector (FLD) that is passed by the labeled glycans is typically a module of the HPLC/LC system.

For the purposes of the present invention, a reference to % will be understood to refer to (v/v) unless explicitly stated otherwise.

As used herein, the term *about* when used together with a numerical value (e.g., a pH value or a percentage value) is intended to encompass a deviation of 20%, preferably 10%, more preferably 5%, even more preferably of 2%, and most preferably of 1% from that value. When used together with a numerical value it is at the same time to be understood as individually disclosing that exact numerical value as a preferred embodiment in accordance with the present invention.

As used herein, the term *comprising* is to be construed as encompassing both "including" and "consisting of", both meanings being specifically intended, and hence individually disclosed, embodiments in accordance with the present invention.

### Further Aspects of the Diclosure

It will be appreciated that the labelling with 2-AA in accordance with the methods and uses described and/or claimed herein is particularly advantageous for mapping (i.e., analyzing) neutral or acidic glycans of a glycoprotein, such as an antibody or erythropoietin. The glycans to be analyzed are preferably N-glycans, particularly N-glycans that are either of the oligomannose, hybrid or complex type. As noted earlier herein, the methods and uses of the present invention *inter alia* have the advantage that they are capable of efficiently distinguishing between the G1F isomers of N-glycans, particularly the G1F isomers with 1,3 or 1,6 galactosylation.

The labeling in accordance with the methods and uses described and/or claimed herein may be done before the glycans have been removed from the glycoprotein. Preferably, however, the labeling is done after the glycans have been removed from the glycoprotein.

Removal of the glycans may be done chemically or enzymatically be methods well known to those skilled in the art.

For example, chemical removal of glycans for subsequent labeling (or analysis, as the case may be) may be effected by hydrazinolysis or by alkaline β-elimination, methods that are well known to those of skill in the art. An exemplary useful kit for chemical removal is the GlycoProfile™ IV Chemical Deglycosylation Kit offered by Sigma-Aldrich.

Removal of the glycans by enzymatic methods is likewise preferred and causes no problems to those of skill in the art. A particularly preferred method of glycan removal for subsequent labeling (or analysis) is digestion with PNGaseF (Peptide N-glycosidase F). Various suitable PNGaseF enzymes are offered commercially under different trade names (*e*.*g*., N-Glycanase®), which are mostly engineered or optimized PNGaseF, although using an engineered or optimized PNGaseF is not mandatory in the context of the present invention. Enzymatic removal may also be done by using Endoglycosidase H (or an enzyme with similar enzymatic activity, such as IgGZERO™) or Endoglycosidase F2, which cleave between the two N-Acetylglucosamines of the glycan core leaving the first monosaccharide attached to the protein. However, the information whether the glycan carried a fucose or not is lost in this way. Furthermore Endoglycosidase H and Endoglycosidase F2 are only specific for oligomannose and hybrid bi-antennary glycans.

The reversed-phase liquid chromatography to be performed in connection with the methods and uses described and/or claimed herein is preferably reversed-phase high performance liquid chromatography (RP-HPLC), or ultra performance liquid chromatography (UPLC). In this case, as in other preferred cases of RP-LC performed in connection with the present invention, these chromatography methods are performed on a reversed-phase liquid chromatography column.

In a preferred aspect of the methods and uses described and/or claimed herein, the mobile phase used during RP-LC comprises formic acid. In this regard, preferred amounts of formic acid in the mobile phases are from about 0.1% to about 2.0% formic acid. Typical preferred amounts therefore include about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, or about 1.9% formic acid. An amount of about 1.0% formic acid in the mobile phase is particularly preferred.

In another aspect of the methods and uses described and/or claimed herein, the mobile phase used during RP-LC comprises acetic acid. In this regard, preferred amounts of acetic acid in the mobile phases are again from about 0.1% to about 2.0% acetic acid. Typical preferred amounts therefore include about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, or about 1.9% acetic acid. An amount of about 1.0% acetic acid in the mobile phase is particularly preferred.

Further, suitable pH values of the mobile phase used during RP-LC are in the range of about 1 to about 4, more preferably in the range of about 1.5 to about 3, yet more preferably of about 1.8 to about 2.9, even more preferably of about 1.9 to about 2.75, and particularly preferably of about 2 to about 2.7. Preferred is in particular a pH value in the range of about 2.1 to about 2.18. Accordingly, preferred mobile phases used during RP-LC in accordance with the methods and uses described and/or claimed herein will have a pH value of about 1.9, 2, 2.1, 2.2, 2.3, 2.4, 2.5, 2.6, or 2.7, with pH values of about 2.1 or of about 2.18 being particularly preferred.

In the course of the methods and uses described and/or claimed herein the separation of the 2-AA labeled glycans by RP-LC may be advantageously performed at temperatures in the range of about 4°C to about room temperature, or at room temperature (with room temperature being defined as 23°C for the purpose of the present invention). Preferably, however, the separation is performed at temperatures above room temperature. Preferred in this regard is a temperature in the range of about 40°C to about 60°C, with about 50°C being particularly preferred.

Suitable flow rates for an RP-LC column in accordance with the present invention will be readily known or determined by those of skill in the art. Generally, suitable flow rates will typically be in a range of about 50-1000 µl per minute. Preferred are, for example, flow rates in a range of about 100-500 µl per minute. Accordingly, preferred values for the flow rate are about 100 µl per minute, about 200 µl per minute, about 300 µl per minute, about 400 µl per minute, or about 500 µl per minute, with a flow rate of about 300 µl per minute being particularly preferred.

As noted above, the methods and uses described and/or claimed herein may comprise subjecting the 2-AA labeled glycans to mass spectrometry (MS) after their separation via RP-LC, with methods where this is done by directly coupling RP-LC with the MS analysis being particularly preferred. Mass spectrometry methods suitable in this regard include ion-trap mass spectrometry, such as positive ionization mass spectrometry.

It will be appreciated that the antibody the glycans of which are analyzed by virtue of the methods and uses described and/or claimed herein may be any type of antibody, as defined above. Particularly preferred are monoclonal antibodies, such as monoclonal antibodies of the IgG type. Thus, a suitable antibody for analysis in accordance with the present invention may be an IgG antibody selected from the group consisting of IgG1, IgG2, IgG3, and IgG4.

The invention is further illustrated by the following Figures and Examples, which are not to be considered as being limiting for the scope of protection conferred by the claims of the present application.

### Examples

### Example 1: Analysis of Glycans Using the 2-AA Method and the 2-AB Method

### Materials

PNGase F was from New England Biolabs. Acetonitrile (ACN) and acetic acid was from Merck. Formic acid and Sodiumcyanoborohydride was from Fluka. PD MiniTrap™ Sephadex® G-10 columns were from GE Healthcare. DMSO was from Sigma. Amicon Ultra 30K filter devices were from Milipore. The mAb glycan standard was prepared at Sandoz. Monoclonal antibodies 1-3 were obtained from in-house development at Sandoz.

### Methods

### Enzymatic N-glycan release using PNGaseF

1 mg of desalted mAb was used. The N-glycans (15 nmol) were released using PNGaseF by incubating the samples overnight (-17 hours) at 37°C. N-glycans were separated from the proteins using Amicon 30K filter devices and were brought to dryness using a speedvac.

### Fluorescence labeling of released N-glycans

Na[BH3(CN)] and either 2-AA or 2-AB were dissolved in 70% DMSO : 30% acetic acid to obtain concentrations of 63 mg/ml and 50 mg/ml, respectively. 15 µl of the labeling solution and 10 µl of deionized water were added to approximately 15 nmol dryed glycans. Labeling reaction was performed at 37°C for 17 h.

Excess label was removed by gel filtration using G-10 columns. Conditioning of the columns was done with 10 ml H₂O. Samples were filled up to 100 µl with deionized water and the sample was applied to the column. After rinsing the column with 700 µl H₂O the purified fluorescence labeled N-glycans were eluted with 600 µl H₂O.

### Reversed Phase HPLC of labeled N-Glycans

Liquid chromatography was performed using an Agilent 1200 Series chromatograph with a Waters ACQUITY UPLC BEH130 C18 (2.1x150 mm 1.7 µm) column. For 2-AA glycan analytics mobile phase A consisted of 1.0% formic acid in H₂O, mobile phase B consisted of 50% ACN in 1.0% formic acid in H₂O. Oven temperature was 50°C and the flow-rate was 0.30 ml/min. The column was equilibrated with 4% B. After injection of up to 100 µl sample the mobile phase composition was held at 4% B for two minutes. B was then raised in four steps to 28%. First to 10% over 27 minutes, then to 11.5% over 10 minutes, then to 14% over 8 minutes and finally to 28% over 19 minutes. The column was regenerated by raising B to 90% over 4 minutes, followed by an isocratic gradient over 2 minutes. The column was then re-equilibrated at 8% B for 5 minutes. Fluorescence detection was performed with an excitation wavelength of 250 nm and an emission wavelength of 425 nm. For analysis of 2-AB labeled glycans the mobile phase consisted of 0.5% formic acid in H₂0 (mobile Phase A) and 0.5% formic acid and 5% ACN in H₂0 (mobile Phase B). Oven temperature was 40°C and the flow rate was 0.3 ml/min. The column was equilibrated with 25% B. After injection the mobile phase was held at 25% B for 2 minutes, B was increased to 55% over 60 minutes and then increased to 61% over 24 minutes. The composition was held for 2 minutes and then initial condition was reached after 2 minutes and held for additional 5 minutes. Fluorescence detection was performed with an excitation wavelength of 250 nm and an emission wavelength of 428 nm.

### Mass Spectrometry

The HPLC was directly coupled to a 3D ion trap ESI-MS (Bruker AmaZon). The ion trap was operated in Enhanced Resolution Mode with a capillary voltage of 4 kV. The capillary temperature was set to 250°C with a nebulizer pressure of 2 bar and a dry gas flow of 6 l/min. MS² spectra were generated with the Auto MS² mode using Collision Induced Dissociation (CID).

### Results

### LC-MS of 2-AB labeld N-glycans

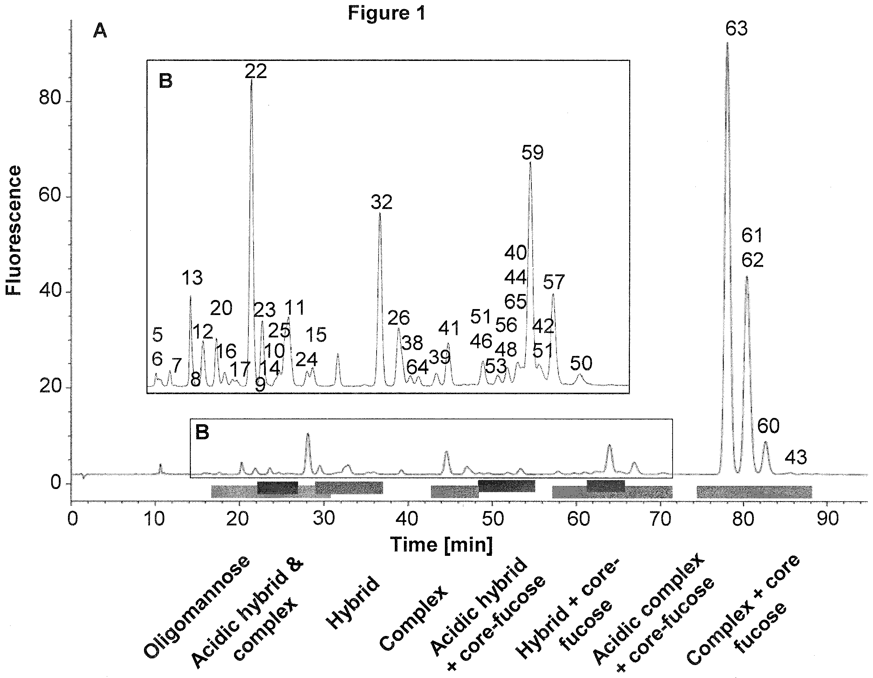

The RP-LC-MS platform used encompassed two structurally closely similar chemical labels, 2-AB and 2-AA. In a first step a RP-LC-MS method for 2-AB labeled N-Glycans was developed. 2-AB labeled N-glycans reveal weak retention properties on C18 columns, therefore a mobile phase gradient with low content of organic solvent was used. The separation was optimal with a mobile phase composition of 0.5% formic acid in water for eluent A and 5% acetonitrile in 0.5% formic in water for eluent B. The resulting fluorescence chromatogram of the 2-AB RP-LC-MS method for the mAb glycan standard is shown in Figure 1. The glycans elute in groups. In order to shorten the run time the mobile phase composition was adapted. The use of formic acid instead of acetic acid improved the retention and led to sharper peaks. A run time of 95 minutes was sufficient to analyze the N-glycans of a mAb.

The first compounds eluting between 16-30 minutes from the column were the high mannose glycans, starting with high to low number of mannose residues (Figure 1). The acidic hybrid and complex glycans (Figure 1) overlapped with the oligomannose group from approximately 22-26 minutes. The next glycans that eluted belonged to the hybrid N-glycans lacking the core-fucose at the terminal GlcNAc (Figure 1), eluting from 28-36 minutes. The complex bi-antennary 2-AB glycans eluted in the middle of the chromatogram (Figure 1, 42-48 min.) right before the acidic hybrid glycans with core-fucose. Hybrid (Figure 1, 57-71 min.) and acidic complex (Figure 1, 48-66 min.) 2-AB glycans, both groups with a fucose residue attached to their core, co-eluted. The group with the most abundant glycans, the complex type glycans with core-fucose, eluted at the end of the chromatogram with a retention time of 74-88 minutes. Sialic acid containing 2-AB labeled glycans eluted in sharp peaks before their corresponding neutral glycans.

Labeled oligomannose and hybrid structures eluted from high to low number of monosaccharides whereas complex type 2-AB glycans, including the acidic variants, eluted from low to high number of monosaccharide units. Several 2-AB glycans eluted with similar retention time and could not be separated. The 2-AB labeled glycans were identified by MS and MS² using the ion-trap mass spectrometer. Table 1 depicted below contains the MS data for the identified glycans and the respective glycan structures are drawn in Figure 3.

**Table 1: 2-AA and 2-AB labeled glycans from mAb glycan standard identified by MS and MS² in the ion-trap. Oberserved and theoretical mass are shown for each assigned glycan. For N-glycan isomers only one mass is shown examplarily. The peak numbers correspond to the appropriate peak numbering in the chromatograms and to the structures in Figure 3.**

| | | 2-AA Glycans | | 2-AB Glycans | |
|---|---|---|---|---|---|
| Peak | Observed Mass | Theoretical Mass | Observed Mass | Theoretical Mass | N-Glycan |
| 5 | 1841.602 | 1841.645 | 1840.689 | 1840.661 | M8 |
| 6,7,8,9 | 1679.541 | 1679.592 | 1678.604 | 1678.608 | M7 |
| 10 | 1031.406 | 1031.381 | 1030.425 | 1030.397 | M3 |
| 11 | 1234.470 | 1234.460 | 1233.489 | 1233.476 | M3G0 |
| 12 | 2027.716 | 2027.709 | 2026.739 | 2026.725 | SM5G1 |
| 13,14 | 1517.592 | 1517.539 | 1516.573 | 1516.555 | M6 |
| 15 | 1396.550 | 1396.513 | 1395.54 | 1395.529 | M3G1 |
| 16 | 1855.655 | 1865.656 | 1865.678 | 1864.672 | SM4G1 |
| 17,18,19 | 1703.619 | 1703.603 | 1702.615 | 1702.619 | SM3G1 |
| 20,21 | 1193.457 | 1193.433 | 1192.49 | 1192.449 | M4 |
| 22 | 1355.525 | 1355.486 | 1354.533 | 1354.502 | M5 |
| 23,24 | 1558.617 | 1558.566 | 1557.582 | 1557.582 | M5G0 |
| 25 | 1720.647 | 1720.618 | 1719.643 | 1719.634 | M5G1 |
| 26,27,28,29 | 1599.618 | 1599.592 | 1598.612 | 1598.608 | G1 |
| 30 | 1720.647 | 1720.618 | 1719.643 | 1719.634 | M6G0 |
| 31 | 1558.592 | 1558.566 | 1557.576 | 1557.582 | M4G1 |
| 32,33 | 1437.586 | 1437.539 | 1436.565 | 1436.555 | G0 |
| 34 | 1687.579 | 1687.608 | - | 1686.624 | SM3G1 |
| 35,36 | 1786.675 | 1786.677 | 1785.708 | 1785.693 | G0F+GN |
| 37 | 1583.710 | 1583.597 | 1582.648 | 1582.613 | G0F |
| 38 | 2173.726 | 2173.767 | 2172.78 | 2172.783 | SM5G1F |
| 39 | 2011.693 | 2011.714 | 2010.754 | 2010.73 | SM4G1F |
| 40 | 2376.763 | 2376.846 | 2375.787 | 2375.862 | SG3F |
| 41 | 1849.705 | 1849.661 | 1848.692 | 1848.677 | SM3G1F |
| 42,43 | 2214.754 | 2214.793 | 2213.818 | 2213.809 | SG2F |
| 44,45 | 2052.732 | 2052.740 | 2051.768 | 2051.756 | SG1F |
| 46 | 1948.741 | 1948.729 | 1947.804 | 1947.745 | G1F+NG |
| 47 | 2028.702 | 2028.729 | - | 2027.745 | M6G1F |
| 48,49 | 1907.689 | 1907.703 | 1906.775 | 1906.719 | G2F |
| 50 | 1866.679 | 1866.676 | 1865.698 | 1865.692 | M5G1F |
| 51,52 | 1704.609 | 1704.624 | 1703.644 | 1703.64 | M5G0F |
| 53 | 1866.650 | 1866.676 | 1865.668 | 1865.692 | M6G0F |
| 54 | 1583.533 | 1583.597 | 1582.608 | 1582.613 | M3G0F+NG |
| 55 | 1704.609 | 1704.624 | 1703.643 | 1703.64 | M4G1F |
| 56,57 | 1542.566 | 1542.571 | 1541.582 | 1541.587 | M3G1F |
| 58 | 2069.748 | 2069.756 | - | 2068.772 | G3F |
| 59 | 1380.553 | 1380.518 | 1379.569 | 1379.534 | M3G0F |
| 60 | 1907.690 | 1907.703 | 1906.746 | 1906.719 | G2F |
| 61,62 | 1745.610 | 1745.650 | 1745.655 | 1744.666 | G1F |
| 63 | 1583.709 | 1583.597 | 1582.607 | 1582.613 | G0F |

### Separation of 2-AA labeled N-glycans

As alternative to the 2-AB labeled glycans also 2-AA labeled glycans were tested with the developed 2-AB method. Most 2-AA glycans remained on the column after the 95 minute gradient, only the oligomannose structures eluted late. Due to this stronger retention of the 2-AA labeled glycans the mobile phase had to be adapted to enable optimal separation. In contrast to 2-AB, 2-AA is negatively charged at neutral pH value. Therefore the composition of the mobile phase was adapted to a lower pH to provide sufficient protons for an efficient ionization in the positive MS mode. At a concentration of 1% formic acid and an ACN content of eluent B increased by a factor of 10 compared to the 2-AB method to 50% optimal separation and good ionization were achieved. The resulting run-time was 78 minutes.

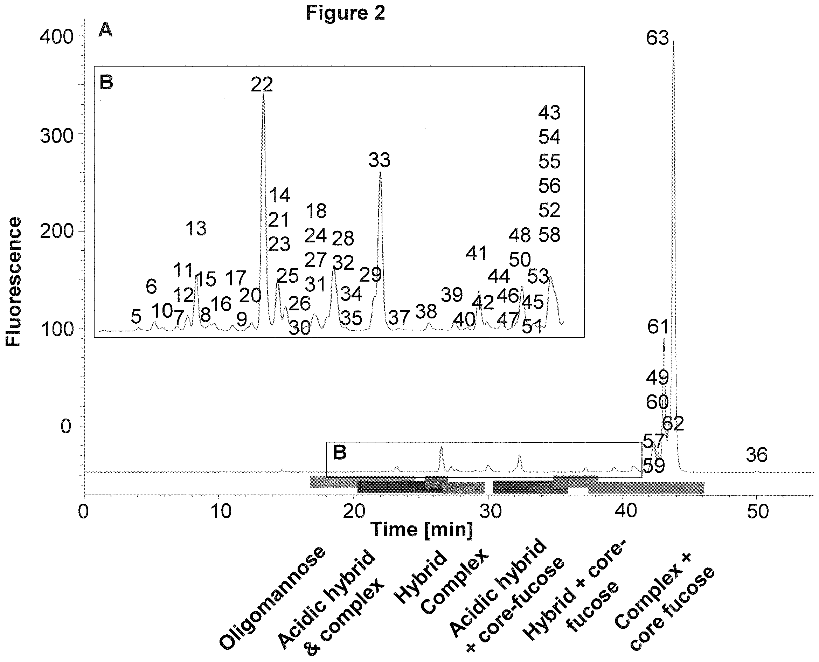

Figure 2 shows the fluorescence chromatogram of the 2-AA labeled mAb glycan standard which is similar to one of the 2-AB method (Figure 1). The labeled glycans elute in different groups, highlighted with different shades of grey in Figure 2. High Mannose structures are the first group eluting from high number to small number of monosaccharide residues, for example from M9 to M5 (19-27 min.). Sialic acid containing non-fucosylated hybrid and complex variants (23-30 min.) are then followed by the neutral non-fucosylated hybrid variants (28-31 min.). Complex structures lacking the core fucose (30-34 min.) elute before sialic acid containing hybrid glycoforms and sialic acid containing complex forms (34-40 min.), both with core fucosylation. Bi-secting structures co-elute with latter. Neutral core-fucosylated hybrid glycans (39-42 min.) elute before the most abundant core fucosylated complex variants in mAbs, the bi-antennary N-glycans (42-51 min.). This grouping is similar to our 2-AB approach and to previous investigations. Sialic acid containing non-fucosylated hybrid and complex variants elute after the high mannose and before the neutral non-fucosylated hybrid group. The same order is observed for the core fucosylated hybrid and complex glycoforms. Again eluting sialic acid containing 2-AA glycans gave the same sharp peak shape as neutral glycans without the need for an ion-pairing reagent. Compared to the 2-AB method, the peaks were sharper and more condensed, but with more overlapping peaks.

### Identification of N-glycans using positive Mode ESI-MS and MS²

The use of 2-AA was so far mostly described for negative ionization MS (Harvey, Journal of Mass Spectrometry: 2005, JMS 40, 642-53; and Prien et al., Analytical Chemistry 2010, 82, 1498-508) due to the negative charge of the acid group. The acidic mobile phase used in this investigation provided good ionization efficiency. 2-AA N-glycans were identified according to their mass derived from MS spectra and by the mass of their appropriate fragments generated by CID in. Table 1 above lists all identified 2-AB and 2-AA N-glycans from the mAb glycan standard with their observed and calculated mass. Some glycan motifs occured several times in course of the chromatogram. These can be structural isomers or bisecting or tri-antennary variants with the same mass that cannot be distinguished as no linkage information is obtained by this LC-MS approach.

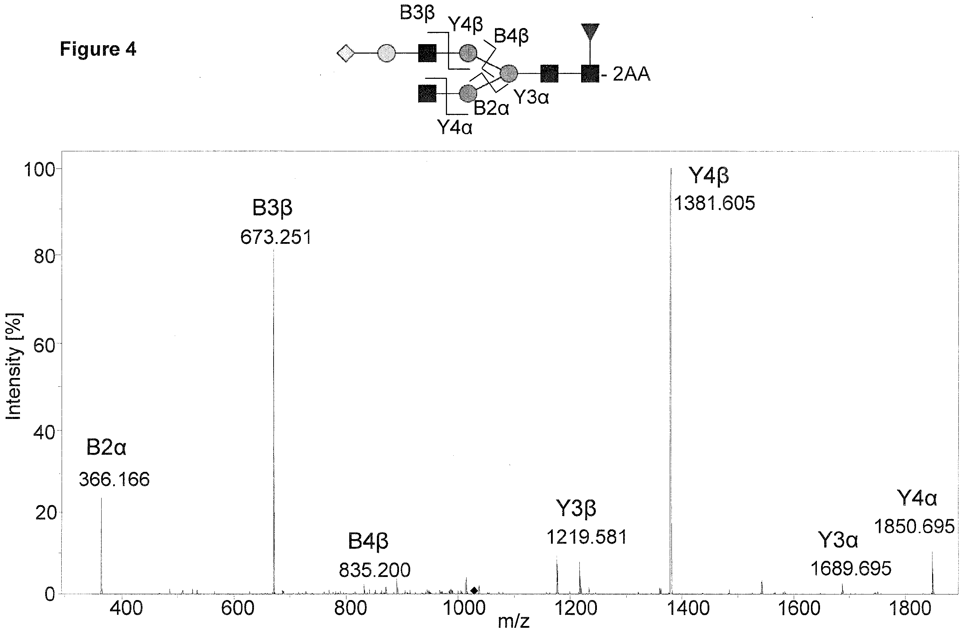

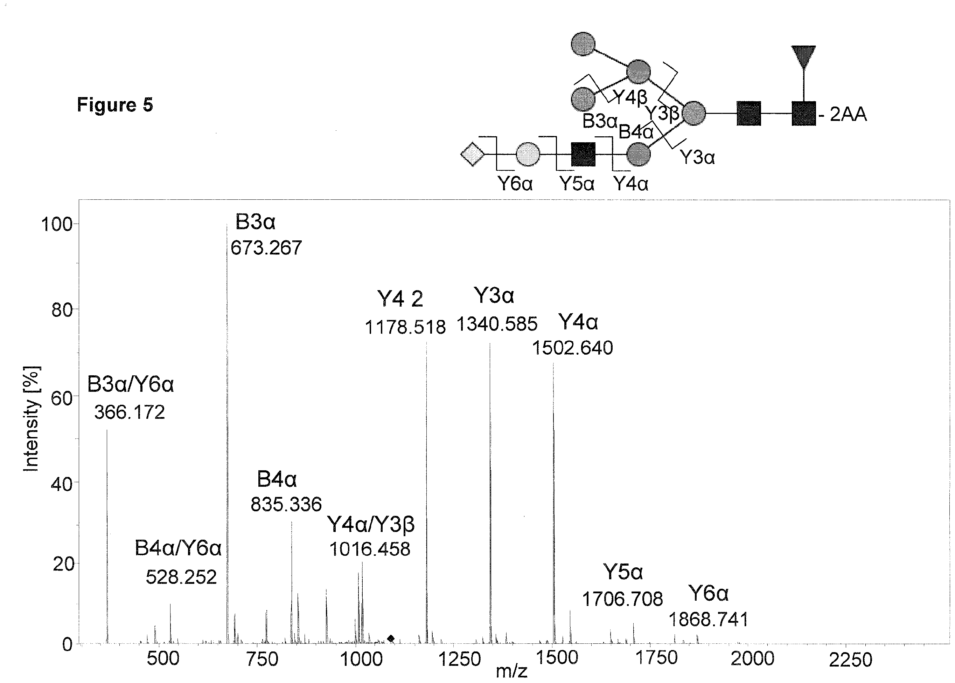

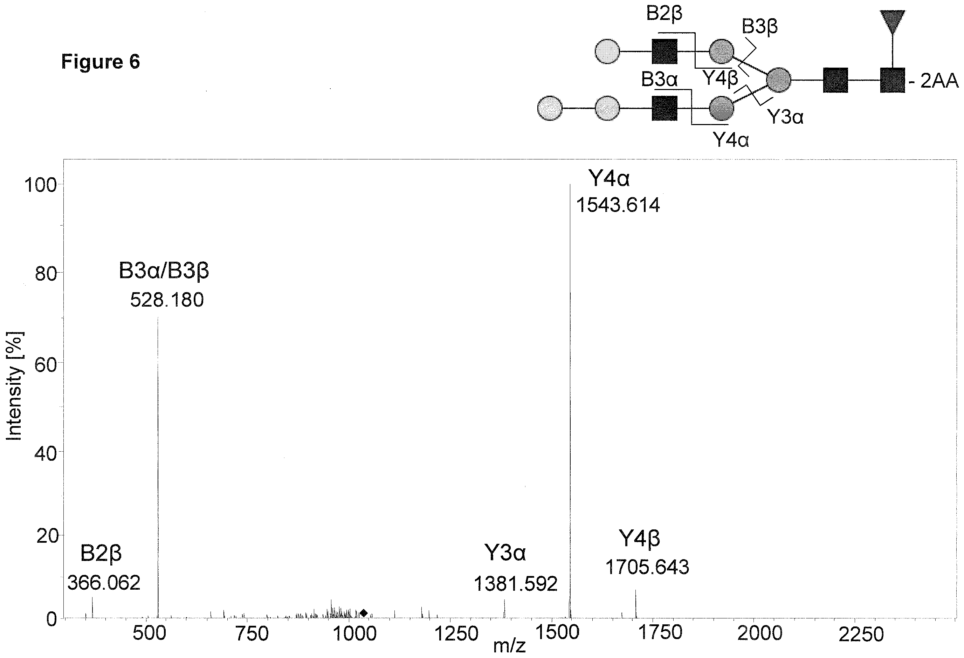

The charge states of the labeled glycans increases with increasing mass. The glycans with a mass < 1200 Da occur almost exclusively as [M+H]¹⁺ ions, whereas glycans with > 1200 Da are double charged [M+2H]²⁺, but also single charged [M+H]¹⁺ ions are present for glycans < 1500 Da. At mass ranges > 1800 Da, the N-glycans begin to ionize as triple charged [M+3H]³⁺ ions with the exception of the oligomannose type glycans which exist at most as double charged ions. Adduct ions are present for all charge states and the oligomannose glycans tend to have the highest affinity for adduct formation. Double and triple charged ions occur as mixed adducts too. [M+Na]¹⁺, [M+K]¹⁺, [M+H+Na]²⁺, [M+2Na]²⁺ and [M+H+K]²⁺ are the most abundant adduct ions. On-line MS detection provides more information than only the identification of N-glycans by their mass and fragments, as co-eluting 2-AA N-glycans can be identified and quantified using the extracted ion chromatograms (EICs) of the appropriate m/z values. Figure 4 and Figure 5 show the MS² spectra obtained after fragmentation of different N-glycoyl-neuraminic acid containing 2-AA glycans. The spectra were acquired on-line using positive ionization ESI-MS and collision induced dissociation (CID) fragmentation. Each of the G1FSG 2-AA glycan fragments (Figure 4) can be explained by the dissociation of a single bond as it is expected using CID in ion-traps. The MS² spectrum at *m*/*z* 1027.9 is dominated by B and Y ions. The sialic acid containing M5G1FSG (Figure 5) shows a slightly different fragmentation pattern. In the MS² spectrum of the [M+2H]²⁺ ion at *m*/*z* 1088.4 only B ions are observed derived from the GlcNAc containing branch, but not from the mannose containing branch of the hybrid structure. The fragments B_{3α}/Y_{6α} with the *m*/*z* 528 and 366 respectively can be explained by the loss of the terminal sialylation. Figure 6 shows the MS² spectrum at *m*/*z* 1035.9 of the rare G3F glycan accounting for less than 0.01% of the glycan pool. B ions are observed for both branches of the glycan since both contain a GlcNAc. This 2-AA glycan co-eluted with the two overlapping and more abundant M3G0F and G2F peaks, but it could be identified and quantified due to on-line MS detection.

### Selectivity of the two developed approaches

The complexity of glycosylation is not only given by the multitude of different N-glycan variants with varying monosaccharide composition. It is also due to the existence of structural isomers with different linkage types. To obtain a glycan-map as comprehensive as possible it is important to separate these isomers.

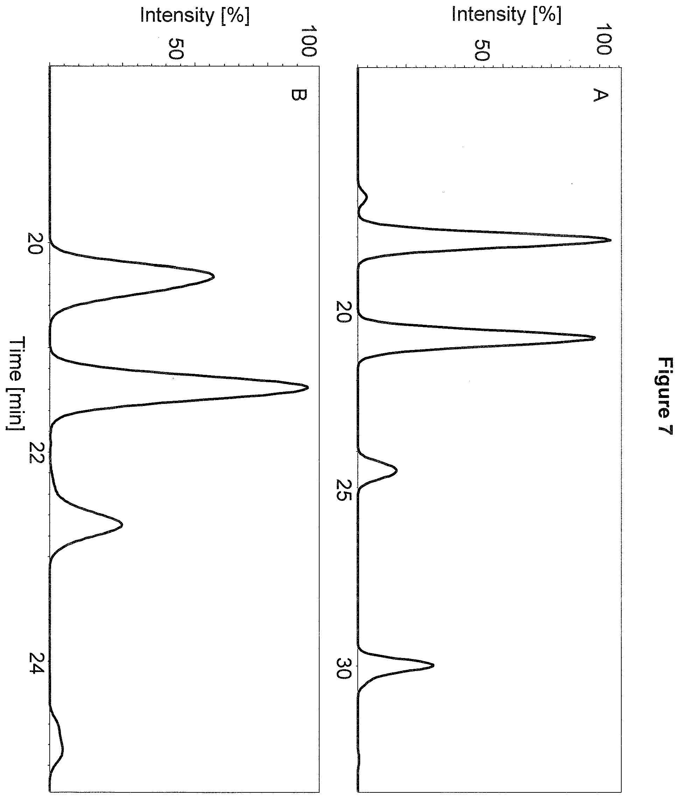

Figure 7 shows the EIC of the M7 isomers of mAb2 for the 2-AB (Figure 7A) as well as for the 2-AA (Figure 7B) labeled glycans. Four different isomers are observed for this high mannose glycan. The linkage could not be deduced with the used reducing end derivatization. The selectivity of the two methods is identical for the high mannose structures only the elution order changed slightly.

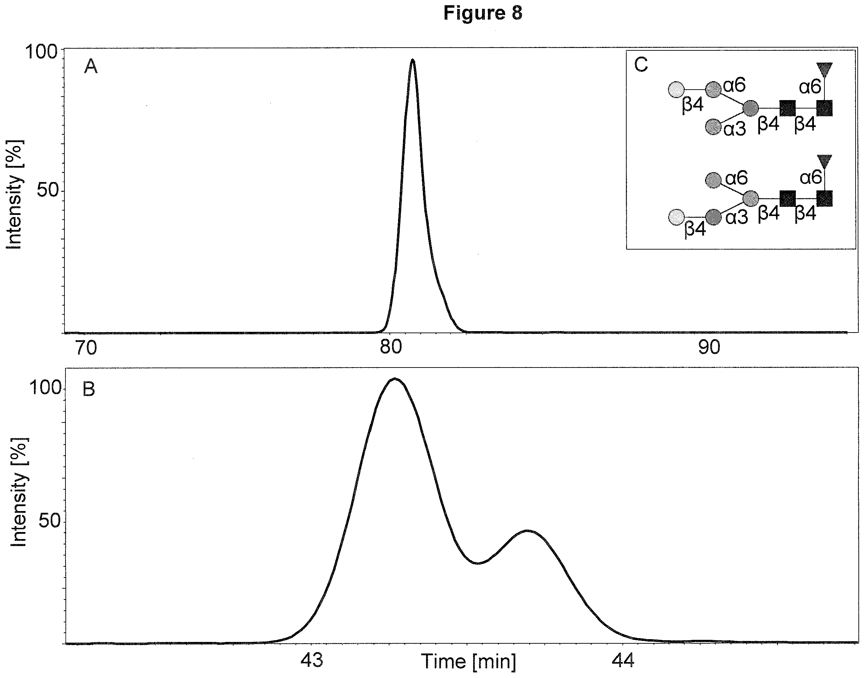

The most abundant structural isomers on mAbs and IgG in general are the G1F isomers (Figure 8C) with the terminal galactose residue at the a1,3 or the a1,6 branch (Flynn et al., Molecular Immunology 2010, 47, 2074-82). So far separation of these two isomers was only described for normal phase and porous graphitized liquid chromatography (Melmer et al., Journal of Chromatography 2011, A 1218, 118-23). In Figure 8 it is clearly shown that the combination of 2-AA as label and the chosen RP-chromatography system is capable of separating these isomers whereas the 2-AB RP-method is not. Figure 8A shows the EIC of the G1F 2-AB glycan which eluted in one peak as a separation could not be achieved throughout the method development. Figure 8B demonstrates the selectivity of the 2-AA method towards the complex N-glycans. From quantitative data obtained by HILIC chromatography we could deduce that the first, bigger peak is the a1,6 isomer and the second smaller peak corresponds to the a1,3 isomer.

### High sensitivity through high injection volume

The possibility of high injection volumes in RP-LC enables the detection and identification of N-glycan variants which occur only at very low level by fluorescence detection or mass spectrometry. Compared to HILIC where only a few µl of an aqueous sample can be injected this circumstance is of huge advantage as the labeled and purified N-glycans were eluted in 600 µl in the last step of the sample preparation. The sample could be injected without any additional concentration steps. N-glycans accounting for less than 0.01% of the whole mAb N-glycan pool like G3F (shown in Figure 6) could be easily detected and identified by ion-trap MS resulting in a high sensitivity glycan-map of the analyzed mAb.

### Analysis of different glycosylated monoclonal antibodies (mAbs)

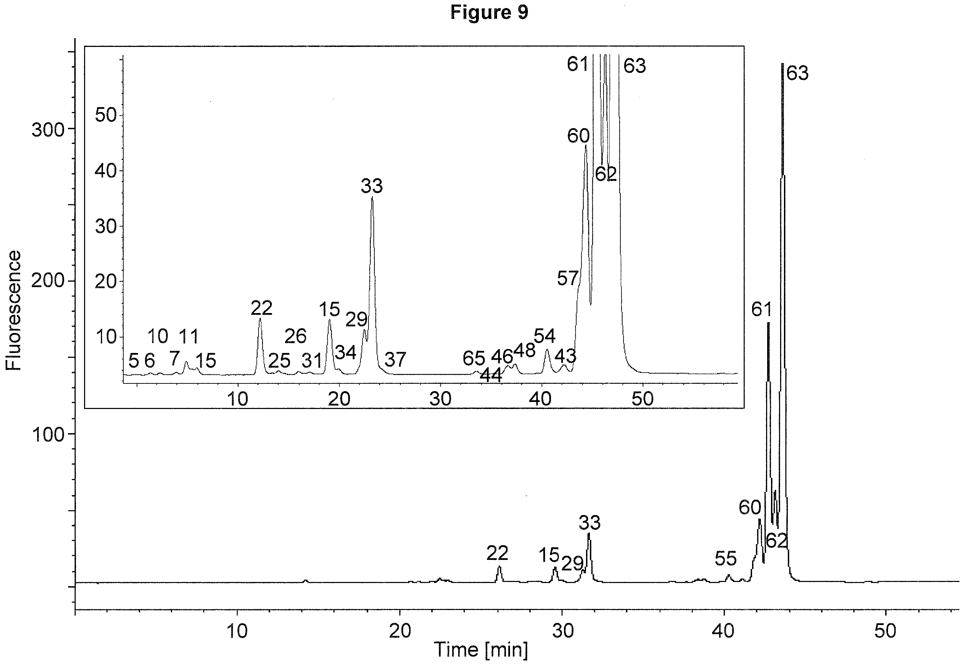

The glycosylation pattern of three different mAbs was analyzed using the developed 2-AA RP-LC-MS method to demonstrate the flexibility and versatility of the method for mAb N-glycan characterization. The fluorescence chromatograms are shown in Figures 9-11. These mAbs were chosen due to their different glycan pool. The N-glycosylation pattern of mAb1 (Figure 9) had the lowest amount of high mannose structures, but the highest portion of non-fucosylated complex glycans. Moreover it was the only mAb with both N-acetyl-neuraminic acid and N-glycoyl-neuraminic acid. It comprised 2% high mannose structures, < 1% hybrid structures without core fucose, and 7% complex structures lacking the core fucose. Hybrid and complex N-glycans with a1,6 core fucose accounted for < 1 % and 90% respectively. About 2% of the N-glycans carried a terminal sialic acid.

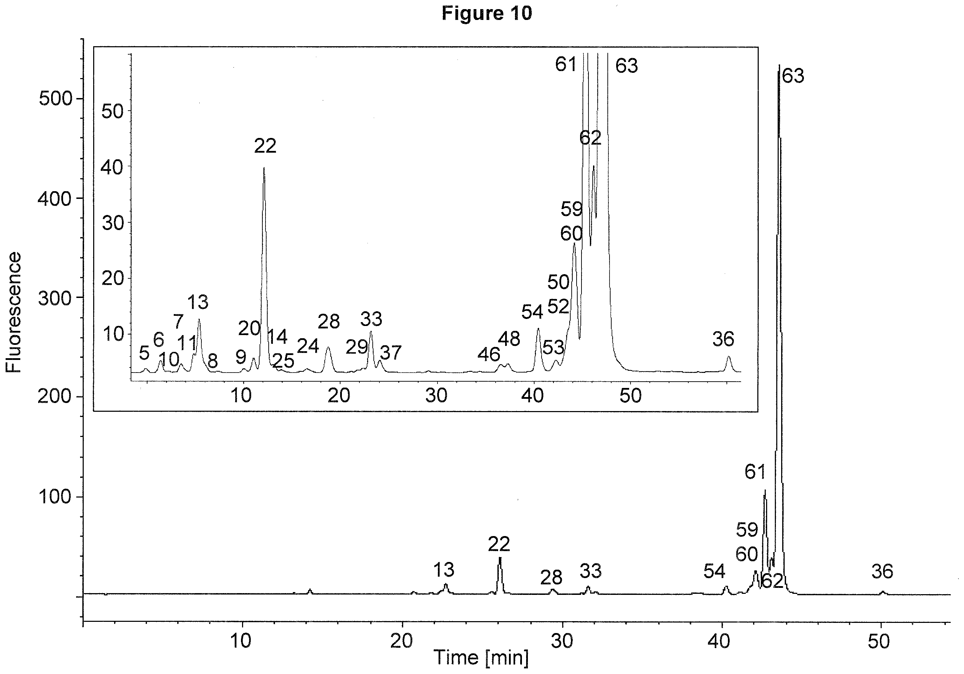

mAb2 (Figure 10) had the highest amount of different oligomannose glycans and carried no sialylation at all. High mannose structures accounted for 7% of all glycans. Non fucosylated hybrid accounted for < 1% and non-fucosylated complex types made 2% of the glycan pool. Hybrid and complex glycoforms with core fucose accounted for 1% and 90%, respectively.

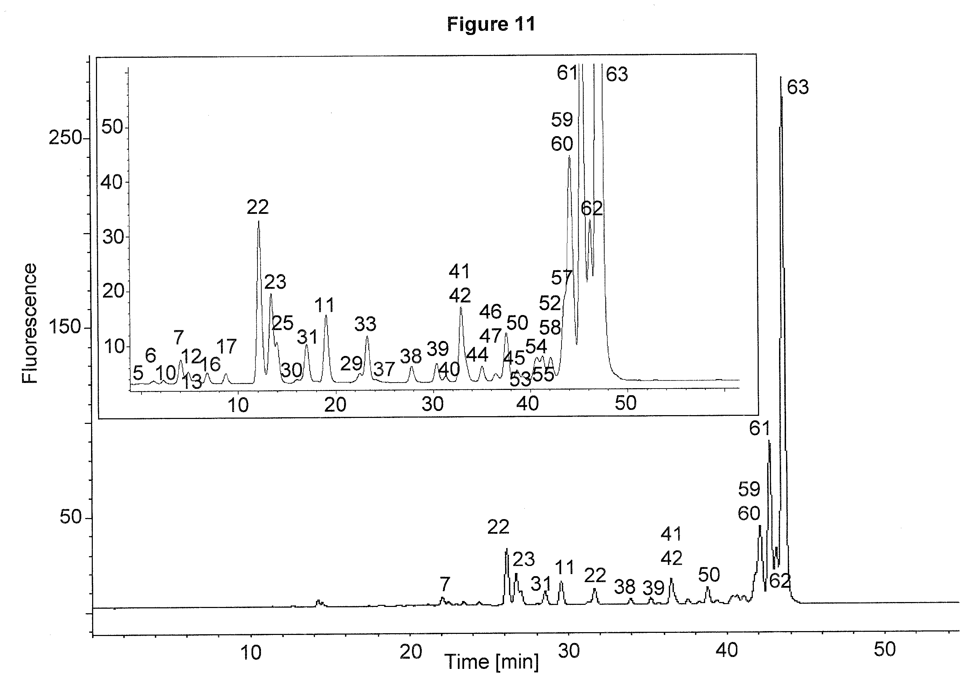

mAb3 (Figure 11) showed the most complex N-glycosylation pattern of the analyzed biopharmaceuticals. It was characterized by a high amount of hybrid structures as well as oligomannose and several sialic acid moieties carrying glycans. The glycosylation comprised 5% oligomannose structures and 5% non-fucosylated hybrid structures. Complex variants accounted for 4% of all glycans. Fucosylated hybrid glycans were present with 5%. Complex fucosylated glycans accounted for 85% and the sialylation level was rather high with 5% for this mAb. The relative glycan composition of the three mAbs was calculated using the peak area of the fluorescence chromatogram of the RP-LC runs, for unresolved or co-eluting glycans the EIC was used for integration. The assigned N-glycan structures to each numbered peak are illustrated in Figure 3.

### Conclusions

The developed glycan map platform according to the disclosure, using RP-HPLC of 2-AB or 2-AA labeled N-glycans, respectively, in combination with fluorescence detection and on-line ion-trap mass spectrometry offers high sensitivity and high resolution for N-glycan analysis. The 2-AA method enables the analysis of neutral and acidic N-glycans with positive ESI MS. Compared to the RP-LC-MS of 2-AB labeled N-glycans more structural isomers can be separated in a shorter analysis time. Especially separation of the highly abundant G1F isomers was achieved. The 2-AA chromatogram of the mAb glycan standard (Figure 2) shows more overlapping peaks or co-eluting peaks respectively which is due to the increased sensitivity and the resulting higher identification rate of the on-line MS detection. The selectivity of the reversed phase chromatography in combination with 2-AA labeling enables separation of various structural isomers from different types of N-glycans and more importantly separates the N-glycans in seven different groups, oligomannose, hybrid and complex without core fucosylation and hybrid and complex with core fucosylation as well as two sialic acid containing groups again with and without the core fucose residue allowing also rapid screening of the glycan composition. The retention of 2-AA on the reversed phase is better compared to 2-AB which may result from a higher hydrophobicity of the protonated carboxyl group of the 2-AA label in the acidic mobile phase. The better retention and separation results in sharper peaks and due to the higher sensitivity of 2-AA in fluorescence detection the analysis of rare glycan species becomes possible. In addition, the focusing of the analyte on the column leads to a more highly concentrated sample entering the ionization chamber of the mass spectrometer and enables efficient ionization and a more sensitive MS detection. Fragmentation of the mostly [M+2H]²⁺ ions by CID produces the mainly occurring B and Y ions providing information about the N-glycan composition. A good ionization of the 2-AA labeled N-glycans was observed in positive ESI MS and even for sialylated structures the MS signal was intensive and on-line MS² data was obtained. Due to the high resolution of the liquid chromatography and the sensitive MS detection, the high complexity of mAb N-glycosylation can be investigated in detail. The high injection volume allows even the detection and quantification of very low abundant N-glycans. The analysis of three mAbs with different glycosylation pattern showed the flexibility of the developed platform for mAb N-glycan characterization. The 2-AA RP-LC/MS approach can be used as a robust and orthogonal method for widely established HILIC of 2-AB glycans or MALDI MS of labeled neutral and acidic N-glycans derived from mAbs and other glycoproteins. With the above experimental data, the strength and versatility of RP-LC combined with on-line MS detection for the analysis of N-glycosylation of glycoproteins is demonstrated.

### Example 2: Use of the 2-AA Method Together with Nano-LC-MS

### Materials

PNGase F was from Roche (Penzberg, Germany). Acetonitrile (ACN) and acetic acid was from Merck (Darmstadt, Germany). Formic acid and Picolineborane were from Fluka (Sigma, Munich, Germany). Protein A sepharose, Protein G sepharose and Sephadex® G-10 96-well plates (custom made) were from GE Healthcare (Munich, Germany). RNase B and DMSO were from Sigma (Munich, Germany). AcroPrep™ Advance Omega™ 10K 96-well filter plates were from Pall (Dreieich, Germany). Human serum was from Lonza. Monoclonal antibody A and cell culture supernatants were obtained from in-house development at Sandoz. G0F glycan standard was from Dextra (MoBiTech, Goettingen, Germany). Complex and acidic N-glycan standards were from TheraProtein (Barcarena, Portugal).

### Methods

### Purification of IgGs from human serum or cell culture supernatant and N-glycan release

Protein G and Protein A sepharose were used to purify IgG from human pooled serum or cell culture supernatant supernatant. A centrifuge with a rotor for 96-well plates (Eppendorf) was used to force liquid through the filter-plates. Serum or cell culture supernatant was applied to a 96-well filter-plate well containing Protein A or Protein G sepharose respectively.

### Enzymatic N-glycan release by use of PNGaseF

After intensive washing with PBS N-glycans were collected and remaining proteins were removed in Pall 10K filter plates and were brought to dryness in a vacuum centrifuge (Christ RVC 2-25). N-glycans were enzymatically released with PNGaseF by incubating the glycoproteins overnight at 37°C in 50 mM phosphate buffer. N-glycans were separated from proteins by use of Pall 10K filter plates and were brought to dryness with the use of a vacuum centrifuge (Christ RVC 2-25).

### Fluorescence labeling of released N-glycans or N-glycan standards

Picolineborane and 2-AA were dissolved in 70% DMSO / 30% acetic acid (v/v) to obtain concentrations of 100 mg/ml and 50 mg/ml, respectively. Labeling solution (15 µl) and deionized water (10 µl) were added to either dried N-glycans or to lyophilized N-glycan standards. Labeling reaction was performed at 37°C for 17 hours.

Excess label was subsequently removed by gel filtration in Sephadex™ G-10 96-well plates. Columns were equilibrated with H₂O (800 µl). Samples were filled up to 100 µl with deionized water and the sample was applied to the column. 2-AA labeled N-glycans were finally eluted with 150 µl H₂O.

### Nano-LC of labeled N-Glycans

Nano-LC (Thermo/Dionex Ultimate 3000) was set-up in "direct injection onto a nano column" mode according to the manufacturer's manual. Column compartment with a RP-column (Dionex Acclaim Pepmap 25 cm; 75 µm I.D.) was held at 40°C. Mobile phase A consisted of 0.5% formic acid. Mobile Phase B consisted of 0.5% formic acid in 50% acetonitrile (ACN). The column was equilibrated with 2% B at a flow rate of 300 nl/min. With a user defined injection routine 1-4 µl sample were stacked between loading solution (0.1% formic acid, 1% ACN in ultrapure water) in a 20 µl sample loop. Sample loop was switched for 5-15 minutes, depending on the injected sample volume, in-line to allow the sample to enter the flow, then the sample loop was switched back to load position to avoid additional gradient delay. Prior to the next injection sample the loop was washed with loading solution. After sample injection, mobile phase B was raised to 30% over 60 minutes, then to 95% over 5 minutes. After holding at 95% B for 5 minutes the column was finally re-equilibrated at 2% B for 15 minutes.

### Mass Spectrometry

Outlet of the nano-LC was directly coupled to an ion trap ESI-MS (Bruker AmaZon) equipped with a recently marketed on-line nano source (Bruker CaptiveSpray®). The ion trap was operated in Enhanced Resolution Mode with a capillary voltage of 1.7 kV. Source temperature was set to 200°C and a dry gas flow of 3 l/min was used to heat the source. MS² and MS³ spectra were generated with the Auto MS² mode, Auto MS³ mode and Collision Induced Dissociation (CID).

### Results

### Method development

N-glycosylation analysis of glycoproteins can be performed on different levels, the intact proteins, proteolytic digests or released glycans can be analyzed by LC-MS. Released glycans can be derivatized to even the ionization efficiency and thereby to improve the accuracy of quantification. This approach provides excellent coverage of the N-glycosylation pattern. As shown herein, 2-AA labeled N-glycans may be identified and quantified by ion-trap MS after RPC. The methods and corresponding uses of the invention are selective for many N-glycan isomers and are robust and reproducible. They are *inter alia* useful to analyze N-glycans of mAbs in advanced stages of development of glycoprotein based drugs. Further, the nano-LC based methods and uses disclosed and claimed herein are particularly useful, e.g., in early stages of development of glycoprotein based drugs, at which attomolar sensitivity may be required.

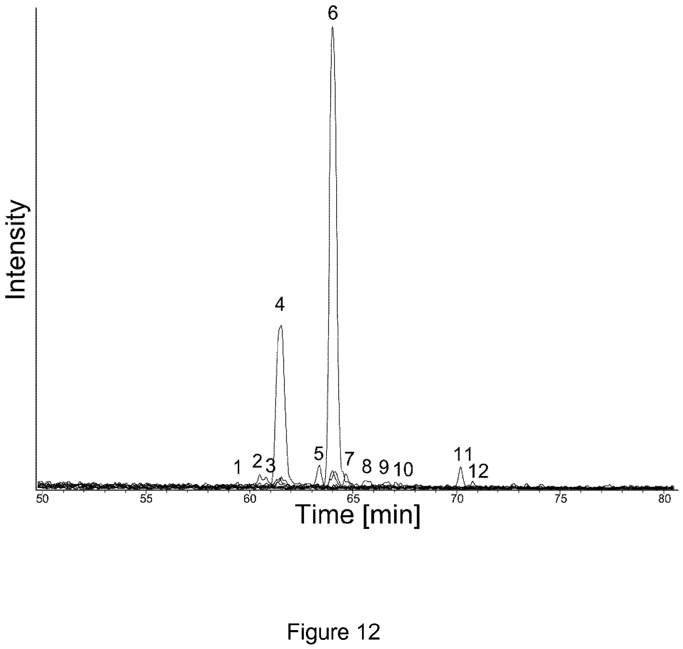

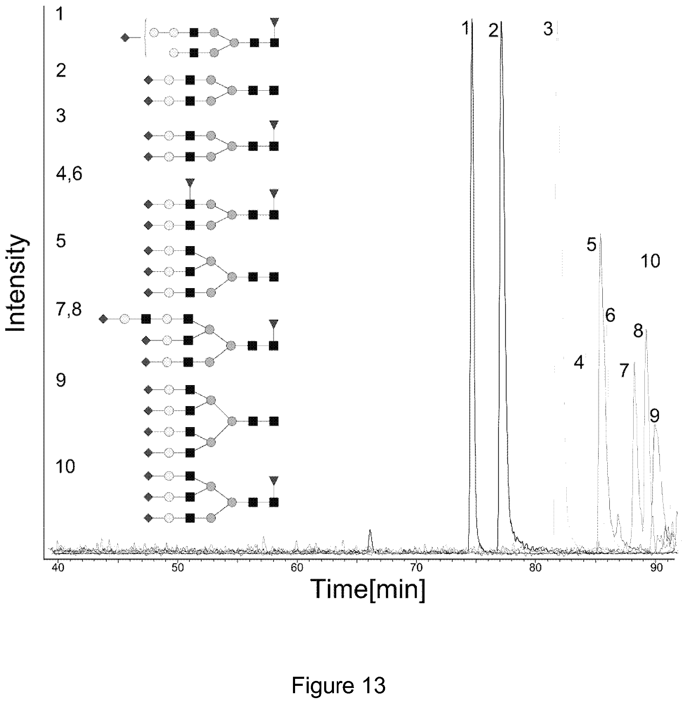

The nano-LC-MS analysis was developed by use of glycans released from RNase B (Figure 12 and Table 2), a model protein for N-glycosylation analysis. RNase B N-glycans were prepared as described in the methods section. Labeled and purified N-glycans were analyzed by nano-LC-MS. The nano-LC was configured in "direct injection on a nano column" mode according to the instruction of the manufacturer, because highly hydrophilic 2-AA N-glycans like the high mannose type glycans did not bind properly onto the trapping column and were therefore underrepresented in setups which included a pre-concentration step with a trapping column. The user defined injection routine allows injection volumes from 1 to 4 µl without major gradient delay by switching the sample loop between 5 to 15 minutes into the flow, depending on the chosen injection volume, to ensure that the entire sample leaves the loop before switching it back to loading position. Because of direct injection, samples have to be highly purified to avoid that salt plugs enter the nanospray chamber of the MS which may damage the emitter tip and shorten its lifetime. The chosen acidic mobile phases resulted in high selectivity for many glycan isomers on RP and improved ionization of glycans in the positive ionization mode. The portion of formic acid in the mobile phase could be lowered to 0.5% compared with 1% for the LC-MS method which may be due to the more efficient ionization in the nanospray. 2-AA glycans occurred mostly as double [M+2H]²⁺ charged ions, smaller N-glycans occurred as single charged ions. Beside protonated ions also mixed adduct ions with sodium (e.g., [M+H+Na]²⁺) or potassium (e.g., [M+H+K]²⁺) were present. 2-AA glycans were identified by MS, MS² and MS³. In addition to RNase B, the method was tested with multiply branched and sialylated glycans to cover all types of glycans. Therefore, several N-glycan standards were labeled with 2-AA and were analyzed (Figure 13). All types of N-glycans can be identified and quantified with this new approach. High mannose glycans elute first, followed by non-fucosylated hybrid and complex variants, then fucosylated hybrid and complex glycans elute. Overall chromatographic resolution is higher for the nano-LC approach in comparison with the LC-MS approach.

**Table 2: RNaseB glycans identified by MS and MS². Figure 12 shows the appropriate chromatogram.**

| **#** | 1 | 2 | 3 | 4 | 5 | 6 | 7 | 8 | 9 | 10 | 11 | 12 |
|---|---|---|---|---|---|---|---|---|---|---|---|---|
| **Glycan** | M8 | M7 | M7 | M6 | M4 | M5 | G2 | M6G0 | M6 | M5G1 | G2F | G1F |

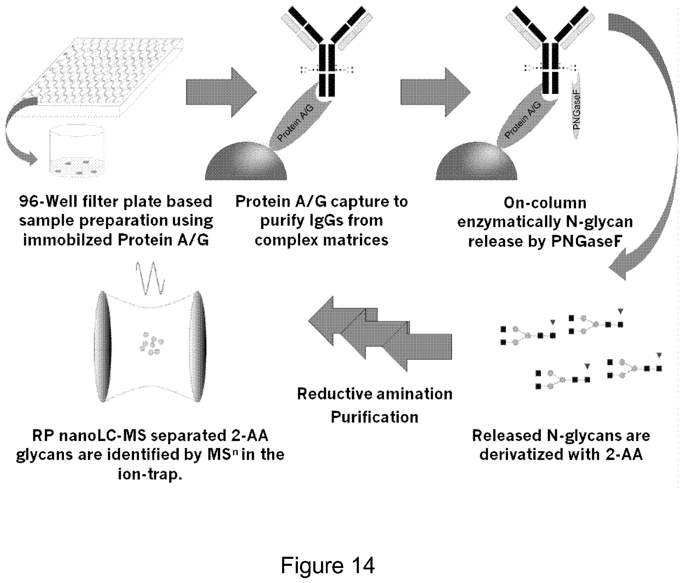

As described above the amount of sample available can be very limited at various stages of biopharmaceutical development especially during early drug development phases like clone selection or pool selection. During clone selection, numerous samples in complex matrices (cell culture supernatant) have to be analyzed which makes affinity purification necessary. Therefore, sample preparation had to be adapted to increase throughput and include affinity purification steps. 96-well plate based sample preparation is a viable option, the success of which has already been demonstrated for glycoprotein analytics (Ruhaak et al., Analytical Chemistry 2008, 80, 6119-26; Wuhrer et al., Proteomics 2007, 7, 4070-81; Reusch et al., Analytical Biochemistry 2013, 432, 82-9). We selected a centrifugation based 96-well filter plate sample preparation as we had observed inhomogeneous flow through the small scale columns in the filter plate wells on a vacuum manifold. Protein A sepharose which is commercially available and state-of-the-art for downstream processing of Fc-part containing biopharmaceuticals (mAbs and fusion proteins) was used as affinity resin. The schematic work-flow is illustrated in Figure 14. Deglycosylation with PNGaseF was performed "on-column". After washing of bound mAb N-glycans were released by incubation of the Protein A-mAb complex with PNGaseF at 37°C followed by elution with water which resulted in higher glycan yields than mAb elution followed by PNGaseF digestion in solution. PNGaseF was subsequently removed by ultrafiltration. Glycans were dried by use of vacuum centrifugation and 2-AA labeling was performed via reductive amination by use of the non-toxic reductive agent picoline borane (Ruhaak et al., Proteomics 2010, 10, 2330-6). Excess label was removed by small scale gel filtration by use of custom made 96-well plates with Sephadex G-10 resin. This last step is a downscaled procedure based on a previously published purification approach (Melmer et al., Analytical and Bioanalytical Chemistry 2010, 905-914).

### Validation of the approach

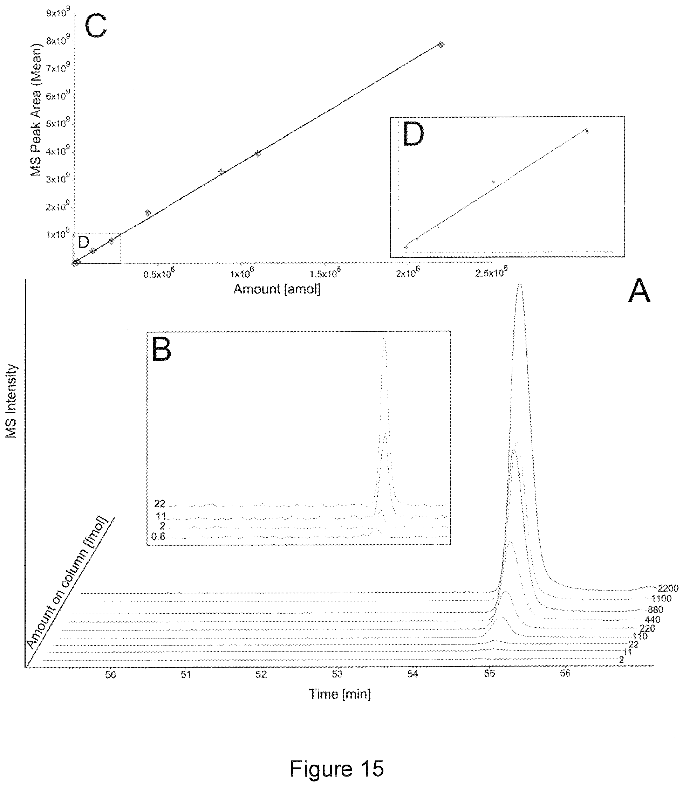

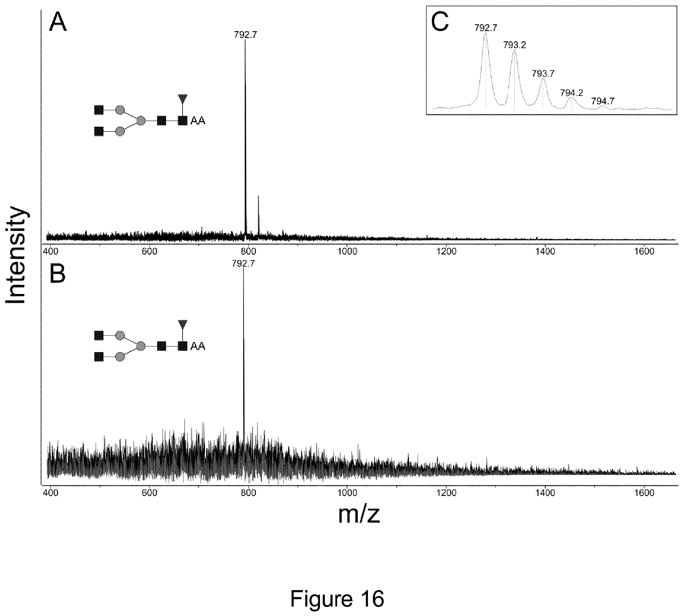

Several parameters, for example sensitivity, robustness, linearity and reproducibility were investigated to validate the nano-LC-MS method. Different column batches were tested and the method was executed on different days and by different operators. To determine overall sensitivity and linearity of the method N-glycan standard G0F was labeled with 2-AA as described in the methods section and serial dilutions were prepared to obtain concentrations ranging from approximately 2.2 pmol/µl to 200 amol/µl. 1 µl of each dilution was injected. EIC peak areas of 2-AA G0F were used for data interpretation (Figure 15A). Example MS spectra are shown in Figure 16. EICs of the four smallest amounts which are still in the linear range are shown in magnified view (Figure 15B). Peak areas were plotted against glycan amount (Figure 15C and Figure 15D) and linearity was evaluated by linear regression with R²=0.9988 indicating good linear correlation between 2.2 pmol and 800 amol. Injections with 600 and 400 amol 2-AA G0F were still detected, but were not in the linear range. 200 amol 2-AA G0F injections were not detected anymore. Thus overall detection limit was approximately 400 amol 2-AA G0F glycan on column and LLOQ (lower limit of quantification) was 800 amol. Next the complete method including sample preparation was validated. Reproducibility was assessed as well as robustness, for example, by comparing results of two different operators (Table 3).

**Table 3: Robustness of the developed approach. Glycan Map of a monoclonal antibody. Sample preparation and nano-LC-MS was performed by two different operators on different days in the same laboratory on the same nano-LC-MS system. For better comparison relative retention times (Rel. RT) based on most abundant G0F are listed. Rel. RT and relative glycan amounts are in good agreement between the two operators.**

| | Operator 1 | | Operator 2 | |
|---|---|---|---|---|
| **Glycan** | **Rel. RT** | **MS %** | **Rel. RT** | **MS%** |
| M7 (1) | 0,77 | 0,11% | 0,78 | 0,10% |
| M7 (2) | 0,78 | 0,15% | 0,79 | 0,10% |
| M6 | 0,80 | 0,58% | 0,81 | 0,64% |
| M7 (3) | 0,81 | <0,10% | 0,82 | <0,10% |
| M7(4) | 0,83 | <0,10% | 0,84 | <0,10% |
| M5 | 0,85 | 2,28% | 0,85 | 2,64% |
| G1 | 0,88 | 0,04% | 0,88 | 0,12% |
| G0 | 0,89 | 0,46% | 0,90 | 0,57% |
| M3 | 0,90 | 0,76% | 0,90 | 0,70% |
| G2F | 0,95 | 0,08% | 0,95 | 0,10% |
| M5G1F/M6G0F | 0,95 | 0,05% | 0,95 | 0,06% |
| G2F | 0,97 | 0,73% | 0,97 | 1,08% |
| G1F | 0,97 | 0,52% | 0,97 | 0,64% |
| M5G1F/M6G0F | 0,98 | 0,15% | 0,98 | 0,20% |
| G1F | 0,98 | 11,48% | 0,98 | 13,27% |
| G1F | 0,99 | 3,42% | 0,99 | 4,10% |
| M3G1F | 0,99 | 1,04% | 0,99 | 1,11% |
| G0F | 1,00 | 74,96% | 1,00 | 71,78% |
| M3G0F | 1,00 | 2,73% | 1,00 | 2,27% |
| G1F+GN | 1,04 | 0,08% | 1,04 | 0,12% |
| G0F+GN | 1,05 | 0,37% | 1,05 | 0,41% |

### Glycan mapping of a monoclonal antibody

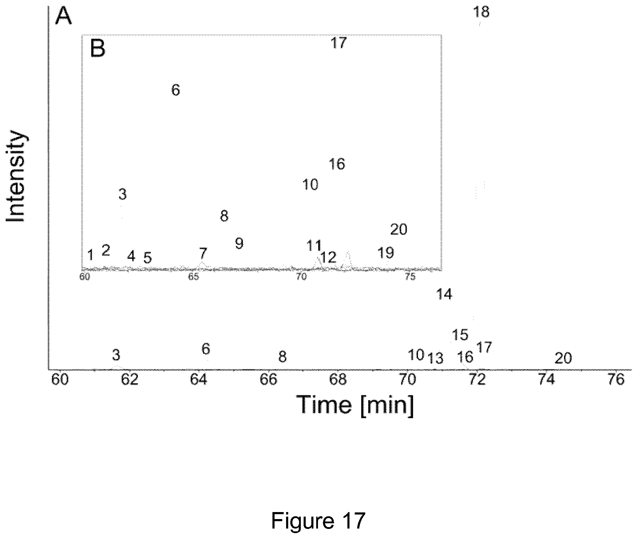

Next we demonstrated the feasibility of the method for characterization of therapeutic glycoproteins. We analyzed the N-glycosylation pattern of a monoclonal antibody. Sample preparation was performed with 10 µg mAb from a drug product formulation. Approximately 1.6 pmol 2-AA labeled N-glycans, corresponding to 800 fmol or 120 ng mAb were finally injected after sample preparation. The resulting EIC is shown in Figure 17. The most abundant oligosaccharides are complex type N-glycans, G0F followed by the two G1F isomers with 1,3 and 1,6 galactosylation respectively. Minor abundant species are shown in magnified view (Figure 17B). Identified N-glycans and relative N-glycan composition of the mAb are listed in Table 4. The absolute N-glycan amounts range from approximately 640 amol for a M7 isomer to 1.1 pmol for the most abundant G0F glycan, again showing the huge differences in relative glycan amounts and the resulting requirements to the method in terms of linearity and sensitivity. Listed structures in Table 4 are deduced from their mass and from MS² as well as from MS³ data. For example, peaks 13, 14 and 15 have the same mass and are not distinguishable by MS² or MS³. Peaks 13 and 14 correspond to G1F with the terminal galactose residue on the 1,3 arm or 1,6 arm respectively and the separated peak 15, also named G1F, might be a truncated bi-secting or tri-antennary variant with the same number of sugar moieties, but not a third G1F isomer. As described above elution order of 2-AA glycans is similar to the pattern obtained by RP-LC-MS. More hydrophilic oligomannose structures elute first followed by non-fucosylated complex variants. Hybrid structures elute before complex type structures as observed for fucosylated glycan structures. Assigned glycan structures are shown in Figure 18.

**Table 4: 2-AA glycans observed in the glycan map of a monoclonal antibody. Glycan structures were identified from their mass and their fragmentation by MS/MS. Theoretical and by ion-trap MS measured values are given. Composition of the N-glycans with their respective portion is depicted on the right.**

| **Glycan map - monoclonal antibody Mass** | | | | |
|---|---|---|---|---|
| **#** | **Measured** | **Calculated** | **Structure** | **Portion** |
| **1** | 1679.45 | 1679.59 | M7 | 0.07% |
| **2** | 1679.46 | 1679.59 | M7 | 0.12% |
| **3** | 1517.53 | 1517.54 | M6 | 0.84% |
| **4** | 1679.47 | 1679.59 | M7 | 0.04% |
| **5** | 1679.46 | 1679.59 | M7 | 0.06% |
| **6** | 1355.46 | 1355.49 | M5 | 2.13% |
| **7** | 1599.49 | 1599.59 | G1 | 0.07% |
| **8** | 1437.48 | 1437.54 | G0 | 0.55% |
| **9** | 1031.34 | 1031.38 | M3 | 0.23% |
| **10** | 1907.57 | 1907.70 | G2F | 0.95% |
| **11** | 1948.57 | 1948.73 | G1F+NG | 0.55% |
| **12** | 1866.53 | 1866.68 | M5G1F | 0.15% |
| **13** | 1745.52 | 1745.65 | G1F | 13.39% |
| **14** | 1745.61 | 1745.65 | G1F | 4.45% |
| **15** | 1745.56 | 1745.65 | G1F | 1.02% |
| **16** | 1542.50 | 1542.57 | M3G1F | 1.02% |
| **17** | 1380.36 | 1380.52 | M3G0F | 2.41% |
| **18** | 1583.58 | 1583.60 | G0F | 71.52% |
| **19** | 1786.57 | 1786.68 | G0F+NG | 0.10% |
| **20** | 1948.59 | 1948.73 | G1F+NG | 0.33% |

### Application during early biopharmaceutical development

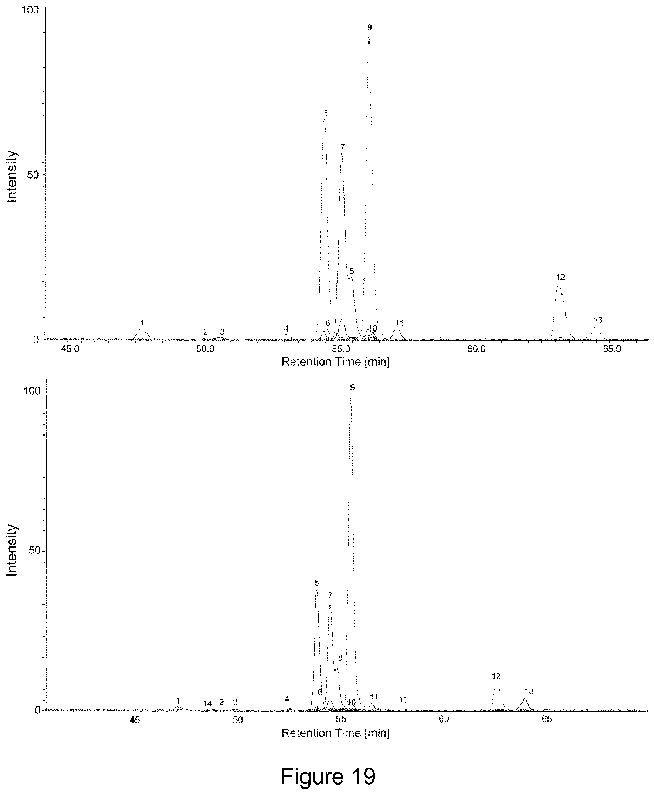

One of the goals was to provide a glycan characterization approach which can be used in a routine manner during clone selection. In early stages of biopharmaceutical development protein producing cells (clones) are cultivated in microtiter plates in a few hundred microliters medium and titers around 1 mg/ml. The aim of this procedure is to identify a clone with appropriate characteristics, for example, distinct protein and glyco variants and a high titer. Clones have to be analyzed comprehensively, but characterization is difficult, because as many different aspects of the biopharmaceutical candidate as possible have to be analyzed, hence only minute amounts can be used for each analysis. With the methods and uses of the present invention, it is possible to characterize N-glycosylation pattern during clone selection employing only minute amounts. Different clones of an Fc-part containing fusion protein from early development were used to demonstrate the feasibility. Cell culture supernatants from different clones with concentrations between 0.3 mg/ml and 1.7 mg/ml were analyzed. Sample preparation was performed with immobilized Protein A as described above and shown in Figure 14. 5 µl supernatant from each clone sample mixed with 45 µl PBS was used. Between 0.27 and 1.5 pmol glycans were injected from each sample depending on the titer. Detailed glycan maps of two different clones based on EICs are shown exemplarily in Figure 19. Assigned glycans and their relative abundance are listed in Table 5. As expected for clone selection samples the N-glycosylation of the two representative clones is similar. Glycans #1-13 are present in both clones, whereas glycoforms 14 (G1) and 15 (G0FB/G0F+GN) are only present for clone 2 with small amounts. Relative distribution of N-glycans is also comparable. Clone 1 has 1.6% oligomannose structures and 10.8% total sialylation and clone 2 has less than 1% oligomannose structures and 8% total sialylation. Terminal galactosylation is slightly different with 60% and 46% for clone 1 and clone 2 respectively. These results demonstrate that this robust nano-LC-MS methodology can be used to characterize N-glycans in very early biotechnological development.

**Table 5: Relative N-glycan composition of two clones from biopharmaceutical development. Chromatograms based on EICs are shown in Figure 19. Glycans 14 (G1) and 15 (G0FB/G0F+GN) are only present in clone 2 (right). Oligomannose content and sialylation levels are highly similar. Terminal galactosylation is slightly different with 60% for clone 1 and 46% for clone 2 respectively.**

| **Glycan Map - Clone 1** | | | **Glycan Map - Clone 2** | | |
|---|---|---|---|---|---|
| **#** | **Glycan** | **MS (Mean %)** | **#** | **Glycan** | **MS (Mean %)** |
| **1** | M5 | 1.64 | **1** | M5 | 0.75 |
| **2** | G0 | 0.12 | **2** | G0 | 0.38 |
| **3** | M3 | 0.32 | **3** | M3 | 0.15 |
| **4** | G1FB/ G1F+GN | 0.52 | **4** | G1FB/ G1F+GN | 0.34 |
| **5** | G2F | 24.79 | **5** | G2F | 18.32 |
| | G0FB/ G0F+GN | 0.85 | **6** | G0FB/ G0F+GN | 1.27 |
| **7** | G1F | 20.57 | **7** | G1F | 15.92 |
| **8** | G1F | 5.69 | **8** | G1F | 5.46 |
| **9** | G0F | 33.11 | **9** | G0F | 47.86 |
| **10** | M3G1F | 0.40 | **10** | M3G1F | 0.22 |
| **11** | M3G0F | 1.26 | **11** | M3G0F | 0.90 |
| **12** | SG2F | 8.83 | **12** | SG2F | 5.74 |
| **13** | SG1F | 1.91 | **13** | SG1F | 2.35 |
| | | | **14** | G1 | 0.23 |
| | | | **15** | G0FB/ G0F+GN | 0.13 |

### Investigation of serum IgG N-glycans

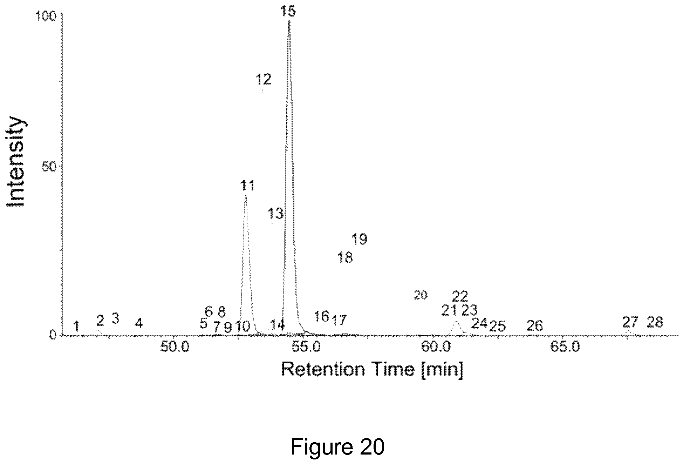

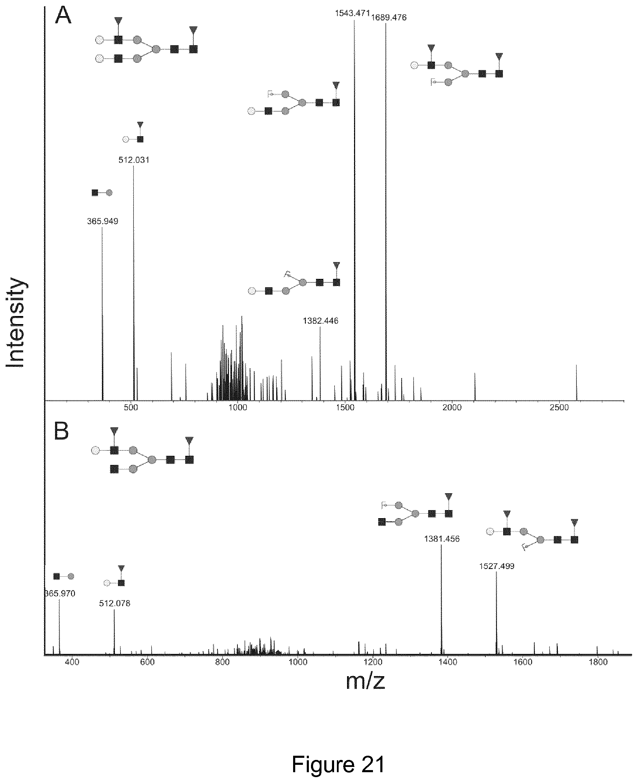

Application of this new approach is not limited to characterization of biopharmaceutical products and product candidates. Due to its high reproducibility application in biomarker discovery investigations is possible. Changes in N-glycosylation of, e.g., IgGs were connected to various diseases and aging in several investigations (Malhotra et al., Nature Medicine 1995, 1, 237-243; Yamada et al., Glycoconjugate Journal 1997, 14, 401-5; Wuhrer et al., Proteomics 2007, 7, 4070-81; Kodar et al., Glycoconjugate Journal 2012, 29, 57-66). As described in the introduction terminal galactosylation or fucosylation influence interactions between IgG molecules and Fc-receptors which in turn affect the immune response. This may influence progression, prognosis or outcome of certain diseases. The methods and uses of the present invention can be used to screen large populations of patients or healthy subjects for significant differences in their respective N-glycosylation. The high resolving power of the nano-LC-MS method and the order of 2-AA N-glycan elution from the RP-column according to their glycan type (e.g., oligomannose, hybrid and complex with and without fucosylation or acidic glycans) allow rapid semiautomatic interpretation of the nano-LC-MS runs. To demonstrate applicability of the method for glycan biomarker discovery, human IgG N-glycosylation was investigated. IgGs were purified from 5 µl of pooled human serum with an estimated IgG concentration of 10 mg/ml with immobilized Protein G sepharose. In contrast to the above described experiments the resin was changed from Protein A to Protein G which allows selective purification of all IgG subclasses 1, 2, 3 and 4. Protein A has no affinity to IgG3 but variable affinity to IgA and IgM and is therefore of limited use for this specific study. A total amount of approximately 4 pmol 2-AA labeled glycans was injected. Resulting chromatograms based on the EICs of the 2-AA N-glycans are shown in Figure 20. A total of 28 different glycoforms were identified and quantified. Detected glycans, relative glycan composition and the retention time relative to most abundant G0F glycans are listed in Table 6, appropriate glycan structures are shown in Figure 18. The majority of the detected N-glycans carries a core fucose and a small portion carries one to two terminal sialic acids. Complex biantennary glycans carrying a core fucose are the most abundant glycans with more than 70%. Double fucosylated glycan species as G1F2 or G2F2 (Figure 21) were also found and were identified by MS² by their signature fragments. These findings correspond to previous reports (Flynn et al., Molecular Immunology 2010, 47, 2074-82).

**Table 6: 2-AA labeled N-glycans identified from human serum IgGs. Assigned N-glycans which were identified by MS, MS² and MS³ are listed. Relative retention time to most abundant G0F and the portion is depicted as well as the portion for each glycoform.**

| **Glycan Map - Human serum IgGs** | | | |
|---|---|---|---|
| **#** | **Rel. Ret.Time** | **Structure** | **MS (Mean %)** |
| **1** | 0.85 | M5 | 0.06% |
| **2** | 0.86 | G2 | 0.52% |
| **3** | 0.88 | G1 | 0.99% |
| **4** | 0.89 | G0 | 0.38% |
| **5** | 0.94 | G3F | 0.01% |
| **6** | 0.94 | G1B/G1+GN | 0.21% |
| **7** | 0.95 | G2F | 0.07% |
| **8** | 0.95 | G0B/G0+GN | 0.12% |
| **9** | 0.95 | G2F2 | 0.01% |
| **10** | 0.96 | G1F2 | 0.01% |
| **11** | 0.97 | G2F | 12.69% |
| **12** | 0.98 | G1F | 22.29% |
| **13** | 0.99 | G1F | 9.31% |
| **14** | 0.99 | SG2 | 0.24% |
| **15** | 1.00 | G0F | 30.08% |
| **16** | 1.02 | G2FB/G2F+GN | 0.82% |
| **17** | 1.03 | G1FB/G1F+GN | 0.43% |
| **18** | 1.04 | G1FB/G1F+GN | 6.38% |
| **19** | 1.05 | G0FB/G0F+GN | 8.09% |
| **20** | 1.09 | SG2F | 3.39% |
| **21** | 1.12 | SG1F | 1.60% |
| **22** | 1.12 | SG2FB | 0.24% |
| **23** | 1.12 | SG1F | 0.23% |
| **24** | 1.12 | SM3G1F | 0.36% |
| **25** | 1.14 | S2G2 | 0.36% |
| **26** | 1.17 | SG1FB | 0.11% |
| **27** | 1.24 | S2G2F | 0.45% |
| **28** | 1.26 | S2G2FB | 0.55% |

With the use of relative retention times and 2-AA N-glycans separated in groups, a rapid screening of the N-glycan type composition can be done semi-automatically by integration of the appropriate region of the chromatogram. For example, the content of sialic acid containing and core fucosylated 2-AA glycans can be quantified by integration of peaks between 1.09 and 1.26 relative retention times. With the same methodology, e.g., the degree of fucosylation can be determined which has been associated with certain types of cancer (Miyoshi et al., Journal of Biochemistry 2008, 143, 725-9). The approach is not limited to IgGs. By adjusting sample preparation other target proteins or protein mixtures can be investigated. These results show that N-glycan biomarker investigation can be performed with the methods and uses of the present invention.

### Conclusions

Starting with RNase B (Figure 12) and several glycan standards (Figure 13), a new method has been developed which enables sensitive and selective analysis of minute glycoprotein amounts with linearity over several orders of magnitude and sensitivity for single glycans in the attomolar range. Compared with a previous publication which reported MS detection of underivatized N-glycans with low femtomolar sensitivity (Wuhrer et al., Analytical Chemistry 2004, 76, 833-8) the methods and uses of the invention have improved sensitivity most probably due to the applied 2-AA labeling. In comparison with the HILIC nano-LC-MS of Kalay et al. (Analytical Biochemistry 2012, 423, 153-162) a shorter run time with equal or even better chromatographic resolving power was achieved with the methods and uses described or claims herein.

With the methods and uses described and/orclaimed herein, many glycan isomers may be differentiated and different kinds of samples may be successfully analyzed with minimal sample consumption. Feasibility of the approach was demonstrated with N-glycan characterization of 160 ng monoclonal antibody from a drug product formulation with high sensitivity. High sample throughput has been achieved with this 96-well plate sample preparation. A previously published highly efficient gel filtration step (Melmer et al., Analytical and Bioanalytical Chemistry 2010, 905-914) has been successfully miniaturized. This purification step desalts 2-AA glycans efficiently and removes excess label in a single step. Together with the demonstrated robustness and reproducibility requirements for routine use in early biopharmaceutical development are fulfilled which is shown with the glycan mapping results of clone selection samples. Minor differences in N-glycosylation of a fusion protein from different clones have been detected which allows early guidance for further development with respect to N-glycosylation.

The ability to identify and quantify all different types of N-glycans including multiply branched and sialylated variants facilitates broad application in different areas of glycobiology. One further possible application has been shown exemplarily with the serum IgG glycan biomarker experiment. Results are in good agreement with a previously published investigation by Flynn et al., Molecular Immunology 2010, 47, 2074-82.

Compared with other recently published high throughput glycan analysis approaches which are also based on 96-well plate sample preparation (Stoeckmann et al., Analytical Chemistry 2013; Burnin et al., Journal of Chromatography. A 2013, 6-11), the methodology described and/or claimed herein using 2-AA instead of 2-AB and RP-nano-LC-MS instead of UPLC or MALDI MS results in an up to ten fold higher sensitivity based on the initial glycoprotein amounts, and a higher glycan coverage of the glycan maps is achieved. To summarize, as shown by the Examples the methods and uses described and/or claimed herein may be practiced in conjunction with RP-nano-LC-MS, and the power of this approach has for N-glycosylation analysis has been clearly demonstrated. In combination with the high-throughput 96-well plate sample preparation procedure, N-glycan characterization can be performed rapidly, with a high sensitivity and in a routine manner during early biopharmaceutical development. The versatility of the methods and uses of the invention was demonstrated with several different possible fields of application.

## Claims

1. A method of analyzing the glycans of a glycoprotein, comprising
(i) labeling the glycans with anthranilic acid (2-AA); and
(ii) separating the labeled glycans using reversed-phase liquid chromatography (RP-LC), wherein
a) the mobile phase used during RP-LC is acidic; and/or
b) the reversed-phase liquid chromatography (RP-LC) is performed under conditions under which the carboxyl group of the 2-AA label attached to the glycans is neutral,
wherein no ion-pairing reagent is used in the mobile phase;
wherein the glycans have been removed from the glycoprotein prior to separating them using RP-LC;
and optionally
(iii) subjecting the labeled glycans to mass spectrometry (MS) after the separation using RP-LC.

2. Use of 2-AA as a label for the glycans of a glycoprotein in the method of analyzing according to claim 1.

3. The method of claim 1 or the use of claim 2, wherein the glycans are neutral or acidic, wherein optionally the glycans are N-glycans of the following types:
(i) oligomannose;
(ii) hybrid; or
(iii) complex;
and wherein further optionally the N-glycans are G1F isomers, particularly the G1F isomers with 1,3 or 1,6 galactosylation.

4. The method or use of any one of the preceding claims, wherein the reversed-phase liquid chromatography is
a) reversed-phase high performance liquid chromatography (RP-HPLC); and/or
b) done on a reversed-phase liquid chromatography column.

5. The method or use of any one of the preceding claims, wherein the mobile phase used during RP-LC comprises
a) formic acid;
b) formic acid in an amount from 0.1% to 2.0%; or
c) formic acid in an amount of 1.0%.

6. The method or use of any one of the preceding claims, wherein
(i) the temperature under which separation is performed is in the range of 40°C to 60°C, or is about 50°C;
(ii) the flow rate is in a range of 100-500 µl per minute, or is 300 µl per minute;
(iii) the mass spectrometry is ion-trap mass spectrometry, particularly positive ionization mass spectrometry; and/or
(iv) the mobile phase has a pH
a) in the range of 1 to 4;
b) in the range of 1.5 to 3;
c) in the range of 1.8 to 2.9;
d) in the range of 1.9 to 2.75
e) in the range of 2 to 2.7;
f) in the range of 2.1 to t 2.18;
g) of 2.1; or
h) of 2.18.

7. The method or use of any one of claims 1-6, wherein the glycoprotein is
a) an antibody;
b) a monoclonal antibody;
c) a monoclonal antibody of an IgG type; or
d) a monoclonal antibody of an IgG type selected from IgG1, IgG2, IgG3, or IgG4; or
e) erythropoietin.

8. The method or use of any one of claims 1 to 7, wherein no ion-pairing reagent is used in the mobile phase.

9. The method or use of any one of the preceding claims, wherein the RP-LC is RP-nano-LC.

10. The method or use of claim 9, wherein the glycoprotein is derived from:
a) one or more protein-producing higher eukaryotic cell clones, wherein optionally the higher eukaryotic cell clone is producing an antibody, or
b) one or more samples from one or more patients or subjects, particularly human patients or subjects, wherein optionally the one or more sample is a human serum sample.

11. The method of any one of claims 9 or 10, wherein the glycoprotein is an antibody and wherein said antibody is adsorbed to a 96-well filter-plate containing Protein A or Protein G sepharose.

12. The method or use of any one of the preceding claims, wherein the method allows for analyzing attomolar concentrations of the 2-AA labeled glycans, e.g., concentrations as low as 800 amol, preferably as low as 600 amol, and more preferably as low as 400 amol.

13. The method or use of any one of the preceding claims, particularly any one of claims 9-12, wherein the method is operated with high throughput.

## Patentansprüche

1. Verfahren zur Analyse der Glykane eines Glykoproteins, das Folgendes umfasst:
(i) Markieren der Glykane mit Anthranilsäure (2-AA);
und
(ii) Abtrennen der markierten Glykane mittels Umkehrphasen-Flüssigchromatographie (RP-LC), wobei
a) die während der RP-LC verwendete mobile Phase sauer ist; und/oder
b) die Umkehrphasen-Flüssigchromatographie (RP-LC) unter Bedingungen durchgeführt wird, unter denen die Carboxylgruppe der an die Glykane gebundenen 2-AA-Markierung neutral ist,
wobei in der mobilen Phase kein Ionenpaarungsreagenz verwendet wird;
wobei die Glykane vor dem Trennen mittels RP-LC aus dem Glykoprotein entfernt wurden;
und wahlweise
(iii) Unterziehen der markierten Glykane einer Massenspektrometrie (MS) nach der Trennung mittels RP-LC.

2. Verwendung von 2-AA als Markierung für die Glykane eines Glykoproteins bei der Analysemethode nach Anspruch 1.

3. Verfahren nach Anspruch 1 oder Verwendung nach Anspruch 2, wobei die Glykane neutral oder sauer sind, wobei die Glykane wahlweise N-Glykane der folgenden Typen sind:
(i) Oligomannose;
(ii) Hybrid; oder
(iii) Komplex;
und wobei ferner die N-Glycane wahlweise G1F-Isomere sind, insbesondere die G1F-Isomere mit 1,3- oder 1,6-Galaktosylierung.

4. Verfahren oder Verwendung nach einem der vorherigen Ansprüche, wobei die Umkehrphasen-Flüssigchromatographie
a) Umkehrphasen-Hochleistungsflüssigchromatographie (RP-HPLC) ist; und/oder
b) auf einer Umkehrphasen-Flüssigchromatographiesäule durchgeführt wird.

5. Verfahren oder Verwendung nach einem der vorherigen Ansprüche, wobei die während der RP-LC verwendete mobile Phase Folgendes umfasst:
a) Ameisensäure;
b) Ameisensäure in einer Menge von 0,1% bis 2,0%; oder
c) Ameisensäure in einer Menge von 1,0%.

6. Verfahren oder Verwendung nach einem der vorherigen Ansprüche, wobei
(i) die Temperatur, unter der die Trennung durchgeführt wird, im Bereich von 40 °C bis 60 °C liegt oder ungefähr 50 °C beträgt;
(ii) die Durchflussrate in einem Bereich von 100-500 µl pro Minute liegt oder 300 µl pro Minute beträgt;
(iii) die Massenspektrometrie eine Ionenfallen-Massenspektrometrie ist, insbesondere positive Ionisationsmassenspektrometrie; und/oder
(iv) die mobile Phase einen pH-Wert aufweist
a) im Bereich von 1 bis 4;
b) im Bereich von 1,5 bis 3;
c) im Bereich von 1,8 bis 2,9;
d) im Bereich von 1,9 bis 2,75;
e) im Bereich von 2 bis 2,7;
f) im Bereich von 2,1 bis 2,18;
g) von 2,1; oder
h) von 2,18.

7. Verfahren oder Verwendung nach einem der Ansprüche 1-6, wobei das Glykoprotein
a) ein Antikörper ist;
b) ein monoklonaler Antikörper;
c) ein monoklonaler Antikörper eines IgG-Typs; oder
d) ein monoklonaler Antikörper eines IgG-Typs, ausgewählt aus IgG1, IgG2, IgG3 oder lgG4; oder
e) Erythropoetin.

8. Verfahren oder Verwendung nach einem der Ansprüche 1 bis 7, wobei kein Ionenpaarungsreagenz in der mobilen Phase verwendet wird.

9. Verfahren oder Verwendung nach einem der vorherigen Ansprüche, wobei die RP-LC RP-Nano-LC ist.

10. Verfahren oder Verwendung nach Anspruch 9, wobei das Glykoprotein abgeleitet ist von:
a) einem oder mehreren Protein produzierenden höheren eukaryotischen Zellklonen, wobei der höhere eukaryotische Zellklon wahlweise einen Antikörper produziert, oder
b) einer oder mehreren Proben von einem oder mehreren Patienten oder Probanden, insbesondere menschlichen Patienten oder Probanden, wobei wahlweise die eine oder mehreren Proben Humanserumproben sind.

11. Verfahren nach einem der Ansprüche 9 oder 10, wobei das Glykoprotein ein Antikörper ist und wobei der Antikörper an einer 96-Mulden-Filterplatte adsorbiert ist, die Protein A- oder Protein G-Sepharose enthält.

12. Verfahren oder Verwendung nach einem der vorherigen Ansprüche, wobei das Verfahren die Analyse attomolarer Konzentrationen der 2-AA-markierten Glykane gestattet, z.B. Konzentrationen von nur 800 amol, vorzugsweise von nur 600 amol und besonders bevorzugt von nur 400 amol.

13. Verfahren oder Verwendung nach einem der vorherigen Ansprüche, insbesondere eines der Ansprüche 9-12, wobei das Verfahren mit hohem Durchsatz betrieben wird.

## Revendications

1. Procédé d'analyse des glycanes d'une glycoprotéine comprenant
(i) le marquage des glycanes avec de l'acide anthranilique (2-AA) ; et
(ii) la séparation des glycanes marqués par chromatographie liquide en phase inverse (RP-LC), dans lequel
a) la phase mobile utilisée pendant la RP-LC est acide ; et/ou
b) la chromatographie liquide en phase inverse (RP-LC) est réalisée dans des conditions dans lesquelles le groupe carboxyle du marqueur 2-AA fixé aux glycanes est neutre,
dans lequel aucun réactif d'appariement d'ions n'est utilisé dans la phase mobile ;
dans lequel les glycanes ont été retirés de la glycoprotéine avant la séparation par RP-LC ;
et facultativement
(iii) la soumission des glycanes marqués à une spectrométrie de masse (MS) après leur séparation par RP-LC.

2. Utilisation de 2-AA en tant que marqueur pour les glycanes d'une glycoprotéine dans le procédé d'analyse selon la revendication 1.

3. Procédé selon la revendication 1 ou utilisation selon la revendication 2, dans lequel les glycanes sont neutres ou acides, dans lequel facultativement les glycanes sont des N-glycanes des types suivants :
(i) oligomannose ;
(ii) hybride ; ou
(iii) complexe ;
et dans lequel en outre facultativement les N-glycanes sont des isomères G1F, particulièrement les isomères G1F avec une 1,3 ou 1,6 galactosylation.

4. Procédé ou utilisation selon l'une quelconque des revendications précédentes, dans lequel la chromatographie en phase inverse est
a) une chromatographie liquide haute performance en phase inverse (RP-HPLC) ; et/ou
b) réalisée sur une colonne de chromatographie liquide en phase inverse.

5. Procédé ou utilisation selon l'une quelconque des revendications précédentes, dans lequel la phase mobile utilisée durant la RP-LC comprend
a) de l'acide formique ;
b) de l'acide formique en une quantité comprise entre 0,1 % et 2,0 % ; ou
c) de l'acide formique en une quantité de 1,0 %.

6. Procédé ou utilisation selon l'une quelconque des revendications précédentes, dans lequel
(i) la température à laquelle la séparation est réalisée se trouve dans la plage de 40 °C à 60 °C, ou est d'environ 50 °C ;
(ii) le débit se trouve dans une plage de 100 à 500 µl par minute, ou est de 300 µl par minute ;
(iii) la spectrométrie de masse est une spectrométrie de masse par piégeage d'ions, particulièrement une spectrométrie de masse à ionisation positive ; et/ou
(iv) la phase mobile a un pH
a) dans la plage de 1 à 4 ;
b) dans la plage de 1,5 à 3 ;
c) dans la plage de 1,8 à 2,9 ;
d) dans la plage de 1,9 à 2,75 ;
e) dans la plage de 2 à 2,7 ;
f) dans la plage de 2,1 à 2,18 ;
g) de 2,1 ; ou
h) de 2,18.

7. Procédé ou utilisation selon l'une quelconque des revendications 1 à 6, dans lequel protéine est
a) un anticorps ;
b) un anticorps monoclonal ;
c) un anticorps monoclonal d'un type IgG ; ou
d) un anticorps monoclonal d'un type IgG sélectionné parmi les IgG1, IgG2, IgG3, ou IgG4 ; ou
e) l'érythropoïétine.

8. Procédé ou utilisation selon l'une quelconque des revendications 1 à 7, dans lequel aucun réactif d'appariement d'ions n'est utilisé dans la phase mobile.

9. Procédé ou utilisation selon l'une quelconque des revendications précédentes, dans lequel la RP-LC est une RP-nano-LC.

10. Procédé ou utilisation selon la revendication 9, dans lequel la glycoprotéine est dérivée de :
a) un ou plusieurs clones de cellules eucaryotes supérieurs produisant des protéines, dans lequel facultativement le clone de cellule eucaryote supérieur produit un anticorps, ou
b) un ou plusieurs échantillons d'un ou plusieurs patients ou sujets, particulièrement de patients ou sujets humains, dans lequel facultativement les un ou plusieurs échantillons sont un échantillon de sérum humain.

11. Procédé selon l'une quelconque des revendications 9 ou 10, dans lequel la glycoprotéine est un anticorps et dans lequel ledit anticorps est adsorbé à une plaque de filtration à 96 puits contenant de la protéine A ou protéine G sépharose.

12. Procédé ou utilisation selon l'une quelconque des revendications précédentes, dans lequel le procédé permet l'analyse de concentrations attomolaires de glycanes marqués au 2-AA, par exemple, de concentrations aussi faibles que 800 amol, de préférence aussi faibles que 600 amol, et de manière davantage préférée aussi faibles que 400 amol.

13. Procédé ou utilisation selon l'une quelconque des revendications précédentes, particulièrement l'une quelconque des revendications 9 à 12, dans lequel le procédé est utilisé avec un rendement élevé.
